# EUROPÄISCHE PATENTANMELDUNG

(11) **EP 2 065 386 A1**
(43) Veröffentlichungstag der Anmeldung: **03.06.2009**
(21) Anmeldenummer: 07118818.9
(22) Anmeldetag: 18.10.2007
(51) Int. Cl.: C07D 471/04, C07D 471/10, C07D 498/10, A61K 31/506, A61P 29/00

(54) **CGRP Antagonisten**

(71) Anmelder: Boehringer Ingelheim Pharma GmbH & Co. KG, 55216 Ingelheim am Rhein (DE)
(72) Erfinder: Die Erfindernennung liegt noch nicht vor
(74) Vertreter: Hammann, Heinz

(57) **Zusammenfassung**

Gegenstand der vorliegenden Erfindung sind neue CGRP-Antagonisten der allgemeinen Formel **I** 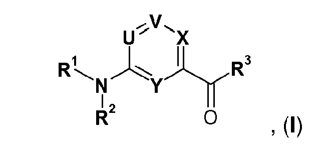 in der **U, V, X, Y, R¹, R²** und **R³** wie nachstehend erwähnt definiert sind, deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen, diese Verbindungen enthaltende Arzneimittel, deren Verwendung und Verfahren zu ihrer Herstellung.

## Beschreibung

Gegenstand der vorliegenden Erfindung sind neue CGRP-Antagonisten der allgemeinen Formel **I** 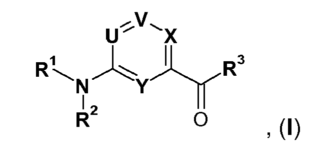 in der **U, V, X, Y, R¹, R²** und **R³** wie nachstehend erwähnt definiert sind, deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen, diese Verbindungen enthaltende Arzneimittel, deren Verwendung und Verfahren zu ihrer Herstellung.

### DETAILLIERTE BESCHREIBUNG DER ERFINDUNG

In der obigen allgemeinen Formel **I** bedeuten in einer ersten Ausführungsform
- **R¹**: eine Gruppe der allgemeinen Formel **IIa** oder **IIb** 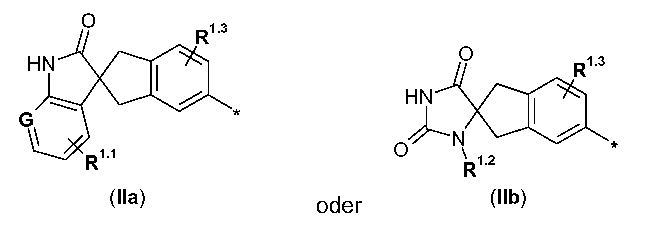
und
- **R²**: H oder C₁₋₃-Alkyl, oder
- **R¹**: und **R²** zusammen mit dem Stickstoffatom, an das sie gebunden sind, eine Gruppe der allgemeinen Formeln **IIIa** oder **IIIb** 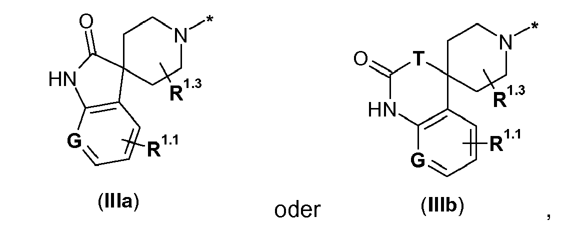
- **G**: C-**R^{1.1}** oder N,
- **T**: N-**R^{1.2}** oder O,
- **R^{1.1}**: unabhängig voneinander (a) H (b) Halogen, C₁₋₃-Alkyl, -OH, -CN, -O-C₁₋₃-Alkyl, -C(O)-O-C₁₋₃-Alkyl, C₂₋₄-Alkenyl, C₂₋₄-Alkinyl, C₁₋₃-Alkyl-S-, Cyclopropyl, -NH₂, -COOH, -NH-C(O)-O-C₁₋₃-Alkyl, -NH-C(O)-C₁₋₃-Alkyl, (c) eine C₁₋₃-Alkyl- oder C₁₋₃-Alkyl-O-Gruppe, in der jede Methylengruppe mit bis zu zwei Fluoratomen und jede Methylgruppe mit bis zu drei Fluoratomen substituiert ist,
- **R^{1.2}**: unabhängig voneinander (a) H oder (b) C₁₋₃-Alkyl,
- **R^{1.3}**: (a) H, (b) F, -CN, C₁₋₃-Alkyl, -CO₂-**R^{1.3.1}** oder (c) eine C₁₋₃-Alkylgruppe, in der jede Methylengruppe mit bis zu zwei Fluoratomen und jede Methylgruppe mit bis zu drei Fluoratomen substituiert sein kann,
- **R^{1.3.1}**: (a) H, (b) C₁₋₆-Alkyl,
- **R³**: eine Gruppe ausgewählt aus
(a) eine mit einem Rest **R^{3.1}** substituierte 5-gliedrige Heteroarylgruppe, die über ein Kohlenstoffatom angebunden ist, ein oder zwei Heteroatome ausgewählt aus der Gruppe N, O und S trägt und mit einer Phenylgruppe oder mit einer 5-oder 6-gliedrigen Carbocyclylgruppe kondensiert ist, wobei die Phenylgruppe zusätzlich mit den Resten **R^{3.2}, R^{3.3}, R^{3.4}** und **R^{3.5}** substituiert ist und die Carbocyclylgruppe zusätzlich mit den Resten **R^{3.2}** und **R^{3.3}** substituiert ist, oder
(b) eine mit einem Rest **R^{3.1}** substituierte 5-gliedrige Heteroarylgruppe, die über ein Kohlenstoffatom angebunden ist, ein oder zwei Heteroatome ausgewählt aus der Gruppe N, O und S trägt und mit einer weiteren 5- bis 6-gliedrigen Heteroaryl- oder Heterocyclylgruppe kondensiert ist, wobei die ankondensierte Heteroarylgruppe mit einem oder zwei Resten **R^{3.2}** substituiert sein kann,
- **R^{3.1}**: (a) H, (b) -C₁₋₆-Alkylen-**R^{3.1.1}**, -C(O)-C₁₋₃-Alkyl,
- **R^{3.1.1}**: (a) H,
(b) -CN, -C(O)-O-**R^{3.1.1.1}**, -O-**R^{3.1.1.1}**, -N(**R^{3.1.1.1}**)₂,
(c) eine 5- bis 7-gliedrige Heterocyclylgruppe, die an einem oder zwei Kohlenstoffatomen mit einem oder zwei Resten **R^{3.1.1.2}** substituiert sein kann,
(d) eine -C(O)-Heterocyclylgruppe, die an einem oder zwei Kohlenstoffatomen mit einem oder zwei Resten **R^{3.1.1.2}** substituiert sein kann,
- **R^{3.1.1.1}**: unabhängig voneinander
(a) H,
(b) C₁₋₆-Alkyl,
- **R^{3.1.1.2}**: unabhängig voneinander
(a) H,
(b) F, C₁₋₃-Alkyl,
- **R^{3.2}**: (a) H,
(b) Halogen, -CN-, OH, -O-C₁₋₃-Alkyl, C₁₋₃-Alkyl, -C(O)-O-**R^{3.2.1}**, -N**R^{3.2.1}R^{3.2.1}**, -NH-C(O)-C₁₋₃-Alkyl, -C(O)-N**R^{3.2.1}R^{3.2.1}**, -S(O₂)-N**R^{3.2.1}R^{3.2.1}**, -O-C(O)-N**R^{3.2.1}R^{3.2.1}**, -N**R^{3.2.1}**-S(O)₂-C₁₋₃-Alkyl, C₂₋₃-Alkenyl, C₂₋₄-Alkinyl, -S-C₁₋₃-Alkyl,
(c) eine C₁₋₃-Alkyl- oder C₁₋₃-Alkyl-O-Gruppe, in der jede Methylengruppe mit bis zu zwei Fluoratomen und jede Methylgruppe mit bis zu drei Fluoratomen substituiert ist,
- **R^{3.2.1}**: unabhängig voneinander
(a) H,
(b) C₁₋₆-Alkyl oder
(c) zwei **R^{3.2.1}** Reste zusammen mit dem Atom, an das sie gebunden sind, auch einen Ring ausbilden können, der ausgewählt ist aus der Gruppe bestehend aus Azetidinyl, Pyrrolidinyl, Piperidinyl, Piperazinyl und Morpholinyl,
- **R^{3.3}**: (a) H,
(b) Halogen, -CN, -OH, -O-C₁₋₃-Alkyl, C₁₋₃-Alkyl, -C(O)-O-**R^{3.3.1}**, -N**R^{3.3.1}R^{3.3.1}**, -NH-C(O)-C₁₋₃-Alkyl, -C(O)-N**R^{3.3.1}R^{3.3.1}**, -S(O₂)-N**R^{3.3.1}R^{3.3.1}**, -O-C(O)-N**R^{3.3.1}R^{3.3.1}**, -N**R^{3.3.1}**-S(O)₂-C₁₋₃-Alkyl, C₂₋₃-Alkenyl, C₂₋₄-Alkinyl, -S-C₁₋₃-Alkyl,
(c) eine C₁₋₃-Alkyl- oder C₁₋₃-Alkyl-O-Gruppe, in der jede Methylengruppe mit bis zu zwei Fluoratomen und jede Methylgruppe mit bis zu drei Fluoratomen substituiert ist,
- **R^{3.3.1}**: unabhängig voneinander
(a) H,
(b) C₁₋₆-Alkyl oder
(c) zwei **R^{3.3.1}** Reste zusammen mit dem Atom, an das sie gebunden sind, auch einen Ring ausbilden können, der ausgewählt ist aus der Gruppe bestehend aus Azetidinyl, Pyrrolidinyl, Piperidinyl, Piperazinyl und Morpholinyl,
- **R^{3.4}**: (a) H,
(b) Halogen, -CN, -OH, -O-C₁₋₃-Alkyl, C₁₋₃-Alkyl, -C(O)-O-**R^{3.4.1}**, -N**R^{3.4.1}R^{3.4.1}**, -NH-C(O)-C₁₋₃-Alkyl, -C(O)-N**R^{3.4.1}R^{3.4.1}**, -S(O₂)-N**R^{3.4.1}R^{3.4.1}**, -O-C(O)-N**R^{3.4.1}R^{3.4.1}**, -N**R^{3.4.1}**-S(O)₂-C₁₋₃-Alkyl, C₂₋₃-Alkenyl, C₂₋₄-Alkinyl, -S-C₁₋₃-Alkyl,
(c) eine C₁₋₃-Alkyl- oder C₁₋₃-Alkyl-O-Gruppe, in der jede Methylengruppe mit bis zu zwei Fluoratomen und jede Methylgruppe mit bis zu drei Fluoratomen substituiert ist,
- **R^{3.4.1}**: unabhängig voneinander
(a) H,
(b) C₁₋₆-Alkyl oder
(c) zwei **R^{3.4.1}** Reste zusammen mit dem Atom, an das sie gebunden sind, auch einen Ring ausbilden können, der ausgewählt ist aus der Gruppe bestehend aus Azetidinyl, Pyrrolidinyl, Piperidinyl, Piperazinyl und Morpholinyl,
- **R^{3.5}**: (a) H,
(b) Halogen, -CN, -OH, -O-C₁₋₃-Alkyl, C₁₋₃-Alkyl, -C(O)-O-**R^{3.5.1}**, -N**R^{3.5.1}R^{3.5.1}**, -NH-C(O)-C₁₋₃-Alkyl, -C(O)-N**R^{3.5.1}R^{3.5.1}**, -S(O₂)-N**R^{3.5.1}R^{3.5.1}**, -O-C(O)-N**R^{3.5.1}R^{3.5.1}**, -N**R^{3.5.1}**-S(O)₂-C₁₋₃-Alkyl, C₂₋₃-Alkenyl, C₂₋₄-Alkinyl, -S-C₁₋₃-Alkyl,
(c) eine C₁₋₃-Alkyl- oder C₁₋₃-Alkyl-O-Gruppe, in der jede Methylengruppe mit bis zu zwei Fluoratomen und jede Methylgruppe mit bis zu drei Fluoratomen substituiert ist,
- **R^{3.5.1}**: unabhängig voneinander
(a) H,
(b) C₁₋₆-Alkyl oder
(c) zwei **R^{3.5.1}** Reste zusammen mit dem Atom, an das sie gebunden sind, auch einen Ring ausbilden können, der ausgewählt ist aus der Gruppe bestehend aus Azetidinyl, Pyrrolidinyl, Piperidinyl, Piperazinyl und Morpholinyl,
- **U**: N, N-Oxid oder CH,
- **V**: N oder C-**R⁴**,
- **X**: N, N-Oxid oder CH,
- **Y**: N oder C-**R⁵**,
wobei höchstens drei der voranstehend genannten Reste **U, V, X** oder **Y** gleichzeitig ein Stickstoffatom darstellen,
- **R⁴**: H, -N**R^{4.1}R^{4.2}** oder -O-C₁₋₃-Alkyl,
- **R^{4.1}**: H oder C₁₋₆-Alkyl,
- **R^{4.2}**: H oder -SO₂-C₁₋₃-Alkyl,
- **R⁵**: H, Halogen oder C₁₋₃-Alkyl,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine weitere Ausführungsform der vorliegenden Erfindung besteht in den Verbindungen der obigen allgemeinen Formel **I**, in denen **U, V, X, Y** und **R³** wie voranstehend unter der ersten Ausführungsform definiert sind und
- **R¹**: eine Gruppe ausgewählt aus 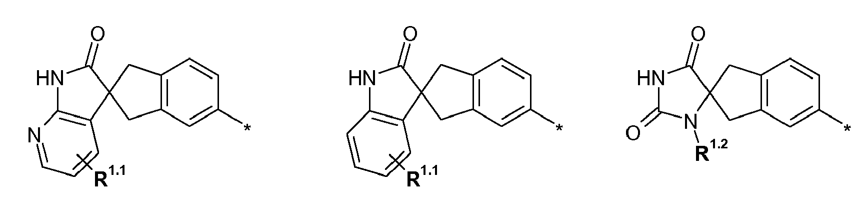 und
- **R²**: H oder
- **R¹** und **R²**: zusammen mit dem Stickstoffatom, an das sie gebunden sind, eine Gruppe ausgewählt aus 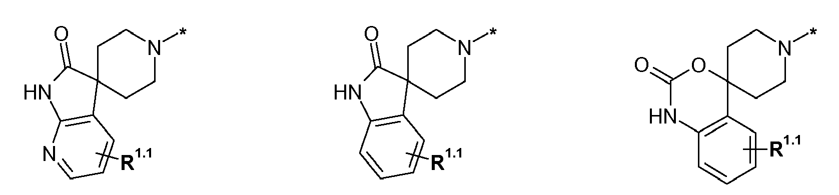 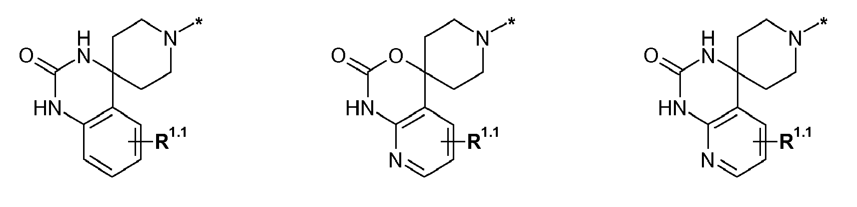
- **R^{1.1}**: unabhängig voneinander
(a) H,
(b) Halogen, C₁₋₃-Alkyl, -OH, -CN, -O-C₁₋₃-Alkyl, -C(O)-O-C₁₋₃-Alkyl, C₂₋₄-Alkenyl, C₂₋₄-Alkinyl, C₁₋₃-Alkyl-S-, -NH₂,
(c) eine C₁₋₃-Alkyl- oder C₁₋₃-Alkyl-O-Gruppe, in der jede Methylengruppe mit bis zu zwei Fluoratomen und jede Methylgruppe mit bis zu drei Fluoratomen substituiert ist,
- **R^{1.2}**: unabhängig voneinander
(a) H oder
(b) CH₃ und
- **R^{1.3}**: (a) H,
(b) F, -CN, -CO₂H, -C(O)-O-C₁₋₃-Alkyl oder
(c) -CF₃ bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine weitere Ausführungsform der vorliegenden Erfindung besteht in den Verbindungen der obigen allgemeinen Formel **I**, in denen **U, V, X, Y** und **R³** wie voranstehend unter der ersten Ausführungsform definiert sind und
- **R¹**: eine Gruppe ausgewählt aus 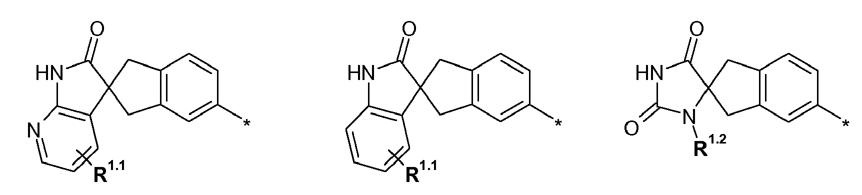 und
- **R²**: H oder
**R¹** und **R²** zusammen mit dem Stickstoffatom, an das sie gebunden sind, eine Gruppe ausgewählt aus 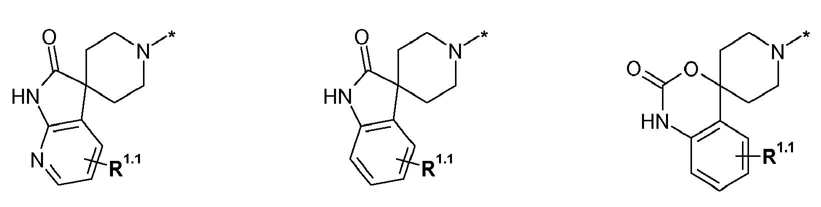 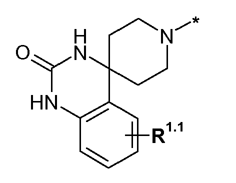
- **R^{1.1}**: unabhängig voneinander
(a) F, CH₃, -OH, -O-CH₃ oder
(b) CF₃ und
- **R^{1.2}**: unabhängig voneinander
(a) H oder
(b) CH₃ bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine weitere Ausführungsform der vorliegenden Erfindung besteht in den Verbindungen der obigen allgemeinen Formel **I**, in denen **U, V, X, Y** und **R³** wie voranstehend unter der ersten Ausführungsform definiert sind und
- **R¹**: eine Gruppe ausgewählt aus 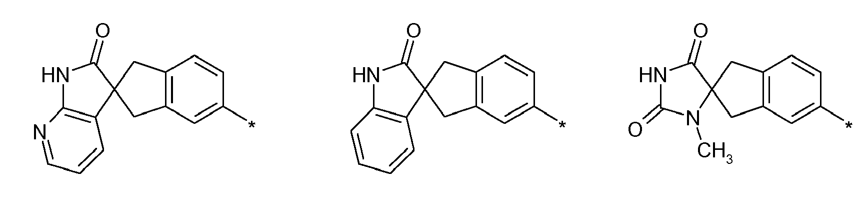 und
- **R²**: H oder
- **R¹** und **R²**: zusammen mit dem Stickstoffatom, an das sie gebunden sind, eine Gruppe ausgewählt aus 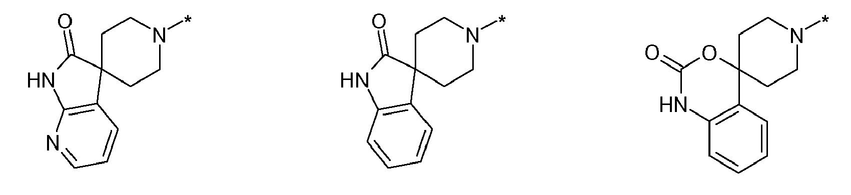 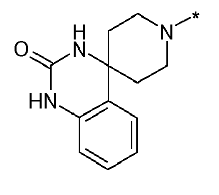
bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine weitere Ausführungsform der vorliegenden Erfindung besteht in den Verbindungen der obigen allgemeinen Formel **I,** in denen **U, V, X, Y, R¹** und **R²** wie voranstehend unter der ersten Ausführungsform definiert sind und
- **R³**: eine Gruppe der allgemeinen Formeln **IV** 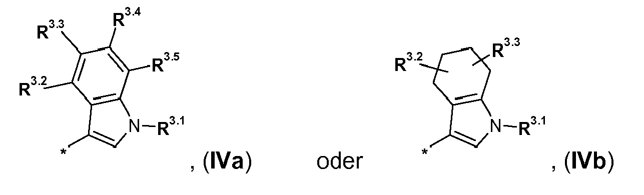
- **R^{3.1}**: (a) H, (b) -C₁₋₆-Alkylen-**R^{3.1.1}**, -C(O)-C₁₋₃-Alkyl,
- **R^{3.1.1}**: (a) H, (b) -C(O)-O-**R^{3.1.1.1}**, -O-**R^{3.1.1.1}**, -N(**R^{3.1.1.1}**)₂, (c) eine 5- bis 7-gliedrige Heterocyclylgruppe, die an einem oder zwei Kohlenstoffatomen mit einem oder zwei Resten **R^{3.1.1.2}** substituiert sein kann, (d) eine -C(O)-Heterocyclylgruppe, die an einem oder zwei Kohlenstoffatomen mit einem oder zwei Resten **R^{3.1.1.2}** substituiert sein kann,
- **R^{3.1.1.1}**: unabhängig voneinander H, C₁₋₆-Alkyl und
- **R^{3.1.1.2}**: unabhängig voneinander H, F, C₁₋₃-Alkyl,
- **R^{3.2}**: (a) H, (b) Halogen, -CN-, OH, -O-C₁₋₃-Alkyl, C₁₋₃-Alkyl, -C(O)-O-**R^{3.2.1}**, C₂₋₃-Alkenyl, C₂₋₄-Alkinyl, -S-C₁₋₃-Alkyl, (c) eine C₁₋₃-Alkyl- oder C₁₋₃-Alkyl-O-Gruppe, in der jede Methylengruppe mit bis zu zwei Fluoratomen und jede Methylgruppe mit bis zu drei Fluoratomen substituiert ist,
- **R^{3.2.1}**: H, C₁₋₆-Alkyl,
- **R^{3.3}**: (a) H, (b) Halogen, -CN, -OH, -O-C₁₋₃-Alkyl, C₁₋₃-Alkyl, -C(O)-O-**R^{3.3.1}**, C₂₋₃-Alkenyl, C₂₋₄-Alkinyl, -S-C₁₋₃-Alkyl, (c) eine C₁₋₃-Alkyl- oder C₁₋₃-Alkyl-O-Gruppe, in der jede Methylengruppe mit bis zu zwei Fluoratomen und jede Methylgruppe mit bis zu drei Fluoratomen substituiert ist,
- **R^{3.3.1}**: H, C₁₋₆-Alkyl,
- **R^{3.4}**: (a) H, (b) Halogen, -CN, -OH, -O-C₁₋₃-Alkyl, C₁₋₃-Alkyl, -C(O)-O-**R^{3.4.1}**, C₂₋₃-Alkenyl, C₂₋₄-Alkinyl, -S-C₁₋₃-Alkyl, (c) eine C₁₋₃-Alkyl- oder C₁₋₃-Alkyl-O-Gruppe, in der jede Methylengruppe mit bis zu zwei Fluoratomen und jede Methylgruppe mit bis zu drei Fluoratomen substituiert ist,
- **R^{3.4.1}**: H, C₁₋₆-Alkyl,
- **R^{3.5}**: (a) H, (b) Halogen, -CN, -OH, -O-C₁₋₃-Alkyl, C₁₋₃-Alkyl, -C(O)-O-**R^{3.5.1}**, C₂₋₃-Alkenyl, C₂₋₄-Alkinyl, -S-C₁₋₃-Alkyl, (c) eine C₁₋₃-Alkyl- oder C₁₋₃-Alkyl-O-Gruppe, in der jede Methylengruppe mit bis zu zwei Fluoratomen und jede Methylgruppe mit bis zu drei Fluoratomen substituiert ist,
- **R^{3.5.1}**: H, C₁₋₆-Alkyl bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine weitere Ausführungsform der vorliegenden Erfindung besteht in den Verbindungen der obigen allgemeinen Formel **I,** in denen **U, V, X, Y, R¹** und **R²** wie voranstehend unter der ersten Ausführungsform definiert sind und
- **R³**: eine Gruppe der allgemeinen Formeln **IV** 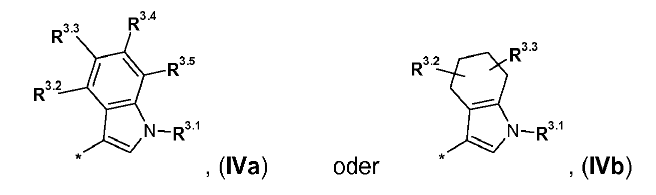
- **R^{3.1}**: (a) H, (b) -C₁₋₄-Alkylen-**R^{3.1.1}**, -C(O)-C₁₋₃-Alkyl,
- **R^{3.1.1}**: (a) H, (b) -C(O)-O-**R^{3.1.1.1}**, -O-**R^{3.1.1.1}**, -N(**R^{3.1.1.1}**)₂, (c) eine 5- bis 7-gliedrige Heterocyclylgruppe, die an einem Kohlenstoffatom mit einem oder zwei Resten **R^{3.1.1.2}** substituiert sein kann, (d) eine -C(O)-Heterocyclylgruppe, die an einem Kohlenstoffatom mit einem oder zwei Resten **R^{3.1.1.2}** substituiert sein kann,
- **R^{3.1.1.1}**: unabhängig voneinander H, C₁₋₆-Alkyl und
- **R^{3.1.1.2}**: unabhängig voneinander H, F, C₁₋₃-Alkyl,
- **R^{3.2}**: (a) H, (b) Halogen, -CN-, -O-C₁₋₃-Alkyl, C₁₋₃-Alkyl, -C(O)-O-**R^{3.2.1}**, (c) eine C₁₋₃-Alkyl- oder C₁₋₃-Alkyl-O-Gruppe, in der jede Methylengruppe mit bis zu zwei Fluoratomen und jede Methylgruppe mit bis zu drei Fluoratomen substituiert ist,
- **R^{3.2.1}**: H, C₁₋₆-Alkyl,
- **R^{3.3}**: (a) H, (b) Halogen, -CN, -O-C₁₋₃-Alkyl, C₁₋₃-Alkyl, -C(O)-O-**R^{3.3.1}**, (c) eine C₁₋₃-Alkyl- oder C₁₋₃-Alkyl-O-Gruppe, in der jede Methylengruppe mit bis zu zwei Fluoratomen und jede Methylgruppe mit bis zu drei Fluoratomen substituiert ist,
- **R^{3.3.1}**: H, C₁₋₆-Alkyl,
- **R^{3.4}**: (a) H, (b) Halogen, -CN, -O-C₁₋₃-Alkyl, C₁₋₃-Alkyl, -C(O)-O-**R^{3.4.1}**, (c) eine C₁₋₃-Alkyl- oder C₁₋₃-Alkyl-O-Gruppe, in der jede Methylengruppe mit bis zu zwei Fluoratomen und jede Methylgruppe mit bis zu drei Fluoratomen substituiert ist,
- **R^{3.4.1}**: H, C₁₋₆-Alkyl,
- **R^{3.5}**: (a) H, (b) Halogen, -CN, -O-C₁₋₃-Alkyl, C₁₋₃-Alkyl, -C(O)-O-**R^{3.5.1}**, (c) eine C₁₋₃-Alkyl- oder C₁₋₃-Alkyl-O-Gruppe, in der jede Methylengruppe mit bis zu zwei Fluoratomen und jede Methylgruppe mit bis zu drei Fluoratomen substituiert ist,
- **R^{3.5.1}**: H, C₁₋₆-Alkyl bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine weitere Ausführungsform der vorliegenden Erfindung besteht in den Verbindungen der obigen allgemeinen Formel **I**, in denen **U, V, X, Y, R¹** und **R²** wie voranstehend unter der ersten Ausführungsform definiert sind und
- **R³**: eine Gruppe ausgewählt aus 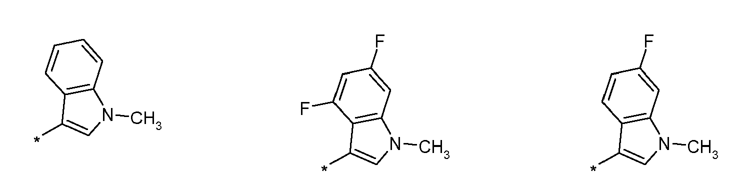 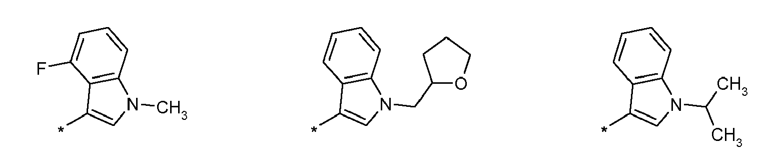 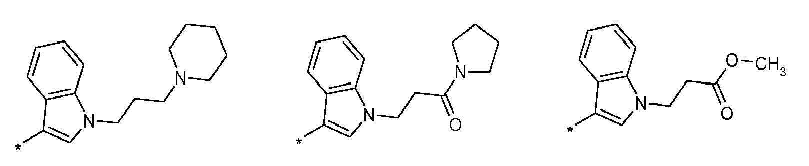 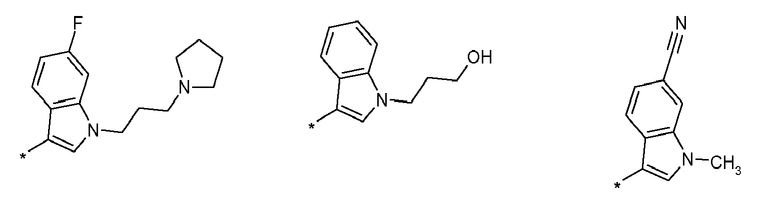 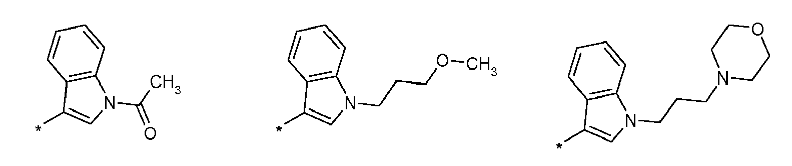 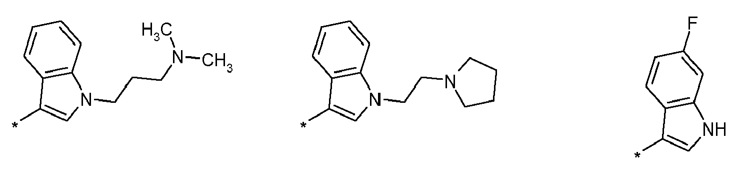 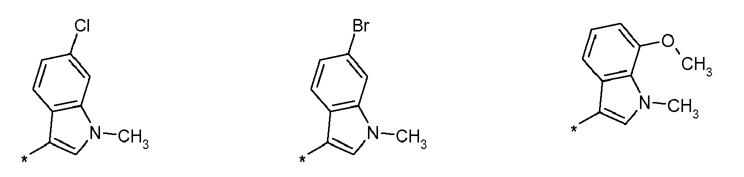 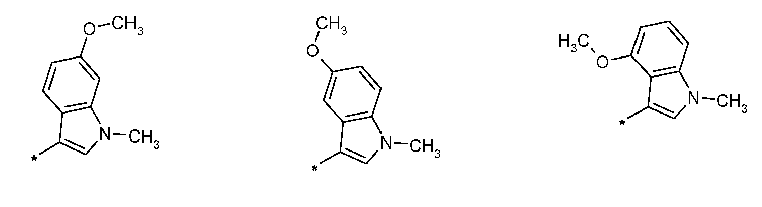 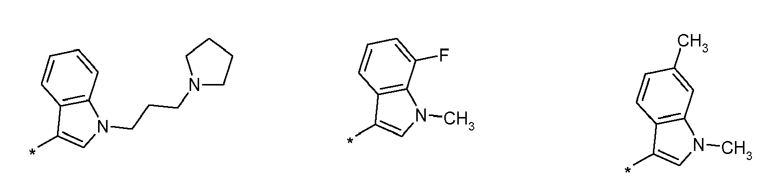 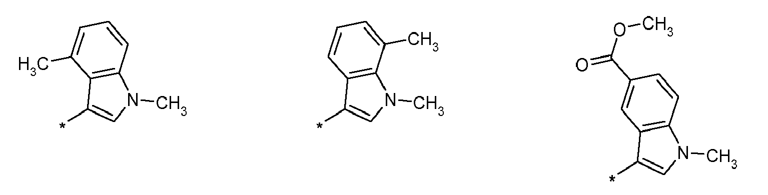 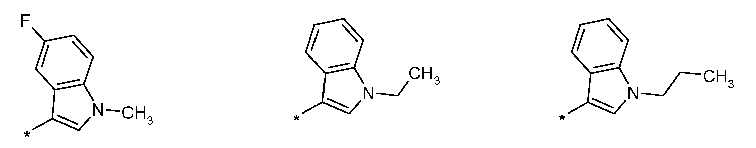 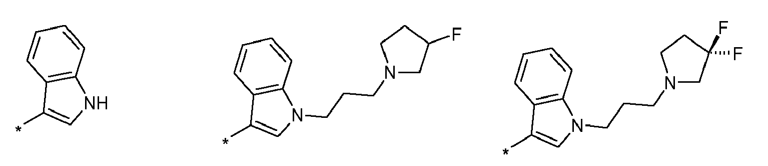 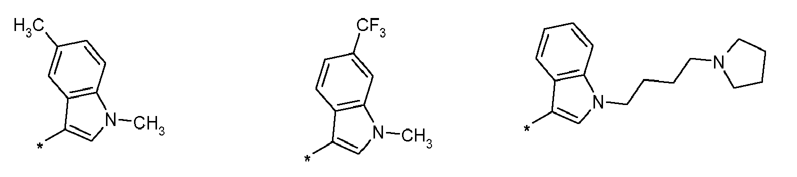 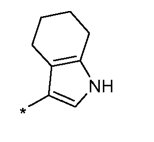
bedeutet, deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine weitere Ausführungsform der vorliegenden Erfindung besteht in den Verbindungen der obigen allgemeinen Formel **I**, in denen **Y, R¹, R²** und **R³** wie voranstehend unter der ersten Ausführungsform definiert sind und
**U-V-X** eine Gruppe ausgewählt aus
-N=N-(CH)=, -N=(C**-R⁴**)-N=, -N=(C-**R⁴**)-(CH)=, -(N-Oxid)=(C-**R⁴**)-(CH)=, -(CH)=N-N=, -(CH)=N-(CH)=, -(CH)=(C-**R⁴**)-N=, -(CH)=(C-**R⁴**)-(N-Oxid)=, -(CH)=(C-**R⁴**)-(CH)= und
- **R⁴**: H, -N**R^{4.1}R^{4.2}** oder -O-C₁₋₃-Alkyl,
- **R^{4.1}**: H oder C₁₋₆-Alkyl,
- **R^{4.2}**: H oder -SO₂-C₁₋₃-Alkyl bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine weitere Ausführungsform der vorliegenden Erfindung besteht in den Verbindungen der obigen allgemeinen Formel **I**, in der
- **R¹**: eine Gruppe ausgewählt aus 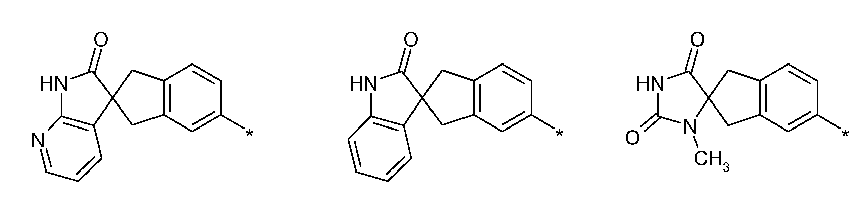 und
- **R²**: H oder
- **R¹** und **R²**: zusammen mit dem Stickstoffatom, an das sie gebunden sind, eine Gruppe ausgewählt aus

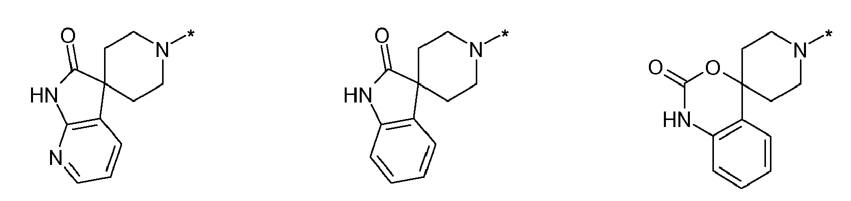

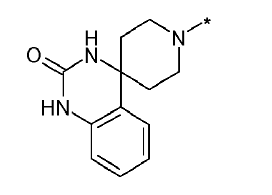

- **R³**: eine Gruppe ausgewählt aus 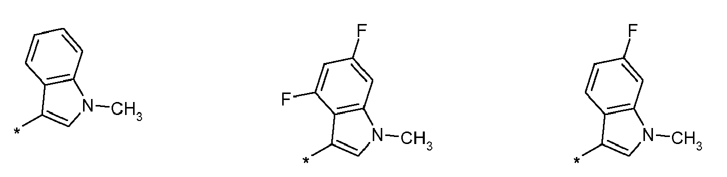 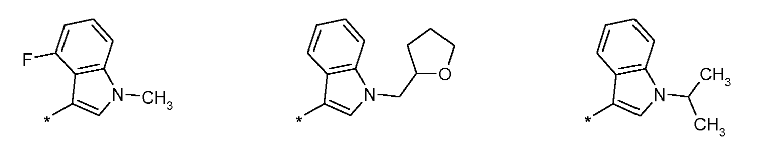 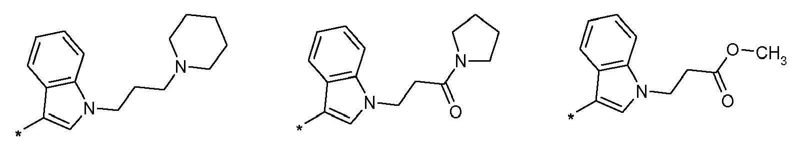 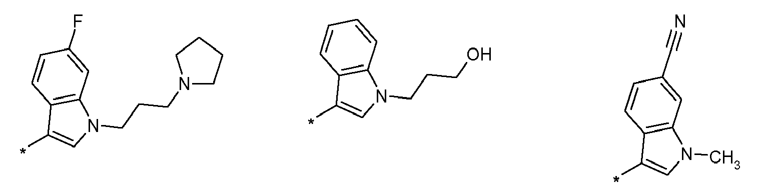 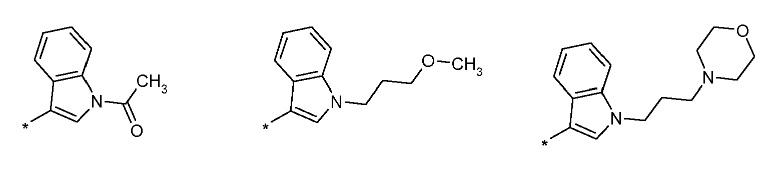 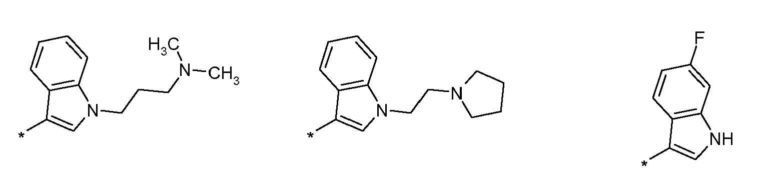 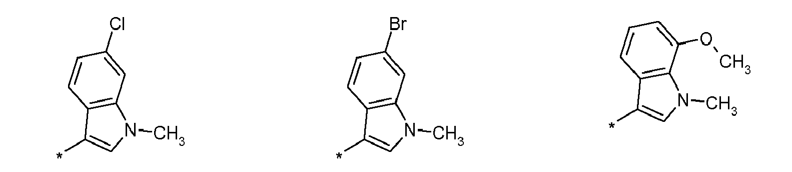 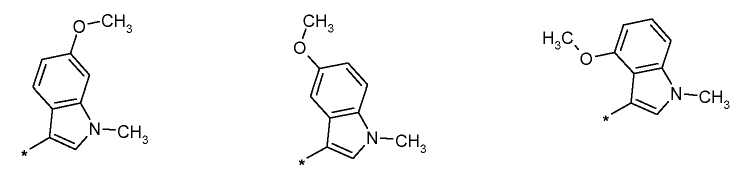 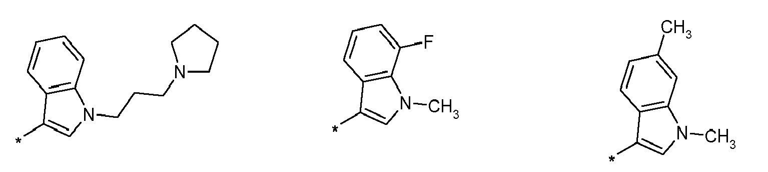 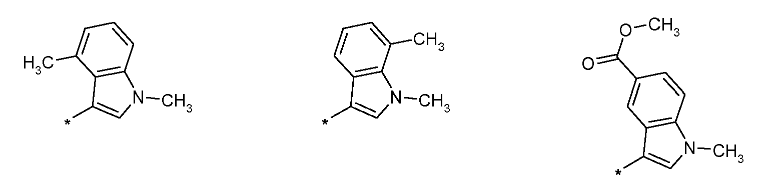 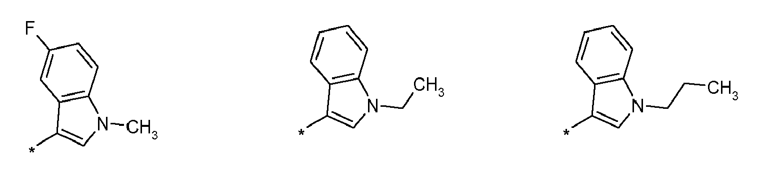 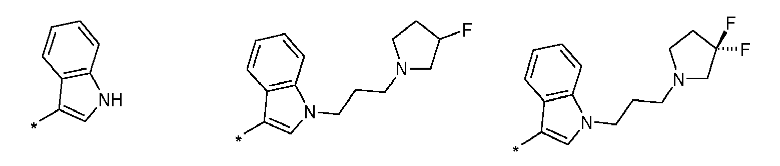 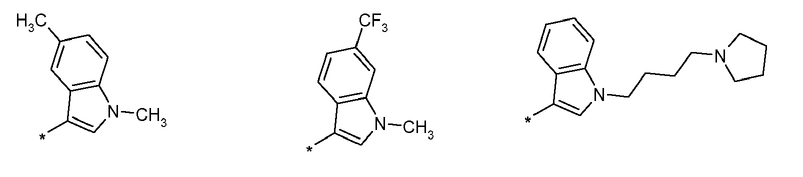 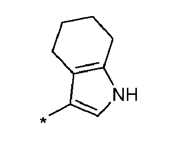
und der Ring 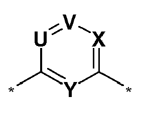 eine Gruppe ausgewählt aus 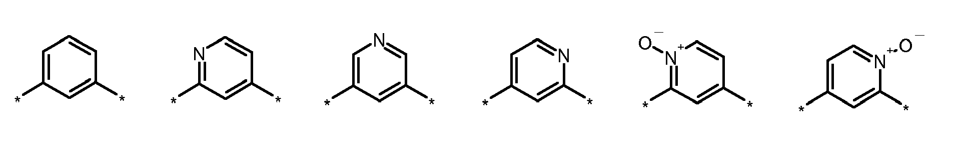 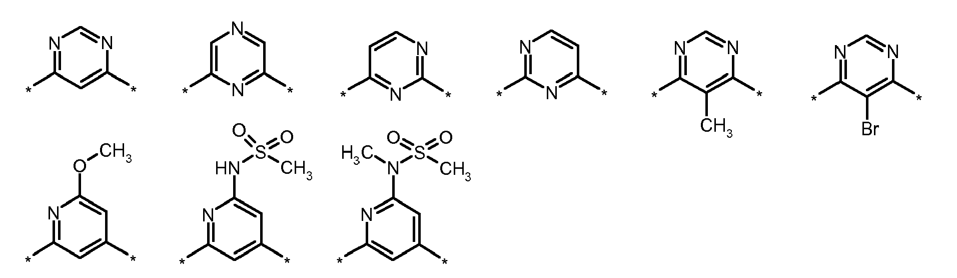 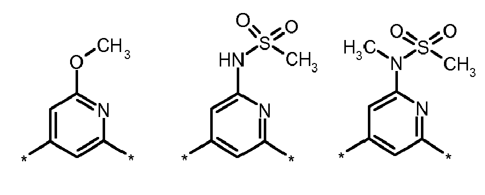 bedeutet, deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Als ganz besonders bevorzugte Verbindungen der obigen allgemeinen Formel **I** seien beispielsweise folgende Verbindungen genannt:

| **Nr.** | **Struktur** |
|---|---|
| (1) | 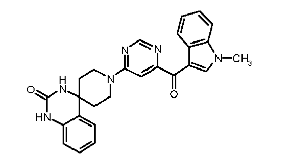 |
| (2) | 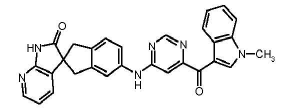 |
| (3) | 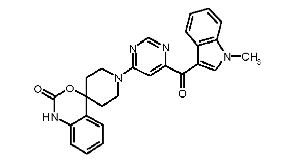 |

deren Enantiomere, deren Diastereomere, deren Hydrate, deren Gemische und deren Salze sowie die Hydrate der Salze, insbesondere deren physiologisch verträgliche Salze mit anorganischen oder organischen Säuren oder Basen.

### VERWENDETE BEGRIFFE UND DEFINITIONEN

Die vorliegende Beschreibung der Erfindung soll in Übereinstimmung mit den Gesetzmäßigkeiten und Regeln der chemischen Bindungen aufgefasst werden. Die Verbindungen, die von dieser Erfindung umfasst sind, sind jene, die auch chemisch stabil sind.
Soweit nicht anders angegeben, sind alle Substituenten voneinander unabhängig. Sollten an einer Gruppe z. B. mehrere C₁₋₄-Alkylgruppen als Substituenten sein, so könnte, im Fall von drei Substituenten C₁₋₄-Alkyl unabhängig voneinander einmal Methyl, einmal Ethyl und einmal *n*-Propyl bedeuten.

Im Rahmen dieser Anmeldung können bei der Definition von möglichen Substituenten, diese auch in Form einer Strukturformel dargestellt werden. Dabei wird, falls vorhanden, ein Stern (*) in der Strukturformel des Substituenten als der Verknüpfungspunkt zum Rest des Moleküls verstanden. Beispielsweise wird ein Phenyl-Rest wie folgt dargestellt: 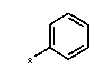 Weiterhin wird das auf den Verknüpfungspunkt folgende Atom des Substituenten als das Atom mit der Positionsnummer 1 verstanden.

Ebenfalls mit vom Gegenstand dieser Erfindung umfasst sind die erfindungsgemäßen Verbindungen, einschließlich deren Salze, in denen ein oder mehrere Wasserstoffatome, beispielsweise ein, zwei, drei, vier oder fünf Wasserstoffatome, durch Deuterium ausgetauscht sind.

Unter dem Begriff "C₁₋₃-Alkyl" (auch soweit sie Bestandteil anderer Reste sind) werden verzweigte und unverzweigte Alkylgruppen mit 1 bis 3 Kohlenstoffatomen, unter dem Begriff "C₁₋₄-Alkyl" werden verzweigte und unverzweigte Alkylgruppen mit 1 bis 4 Kohlenstoffatomen und unter dem Begriff "C₁₋₆-Alkyl" werden verzweigte und unverzweigte Alkylgruppen mit 1 bis 6 Kohlenstoffatomen verstanden. Beispielsweise werden hierfür genannt: Methyl, Ethyl, *n*-Propyl, *iso-*Propyl, *n*-Butyl, *iso*-Butyl, *sec*-Butyl, *tert*-Butyl, Pentyl, Neopentyl oder *n*-Hexyl. Gegebenenfalls werden für vorstehend genannten Gruppen auch die Abkürzungen Me, Et, *n*-Pr, *i*-Pr, *n*-Bu, *i*-Bu, *t*-Bu, etc. verwendet. Sofern nicht anders beschrieben, umfassen die Definitionen Propyl und Butyl alle denkbaren isomeren Formen der jeweiligen Reste. So umfasst beispielsweise Propyl *n*-Propyl und *iso*-Propyl, Butyl umfasst *iso*-Butyl, *sec*-Butyl und *tert*-Butyl etc.

Unter dem Begriff "C₁₋₆-Alkylen" (auch soweit sie Bestandteil anderer Reste sind) werden verzweigte und unverzweigte Alkylengruppen mit 1 bis 6 Kohlenstoffatomen und unter dem Begriff "C₁₋₃-Alkylen" werden verzweigte und unverzweigte Alkylengruppen mit 1 bis 3 Kohlenstoffatomen verstanden Beispielsweise werden hierfür genannt: Methylen, Ethylen, Propylen, 1-Methylethylen, Butylen, 1-Methylpropylen, 1,1-Dimethylethylen, 1,2-Dimethylethylen, Pentylen, 1,1-Dimethylpropylen, 2,2-Dimethylpropylen, 1,2-Dimethylpropylen, 1,3-Dimethylpropylen oder Hexylen. Sofern nicht anders beschrieben, umfasst die Definition Propylen alle denkbaren isomeren Formen der jeweiligen Reste gleicher Kohlenstoffanzahl. So umfasst beispielsweise Propyl auch 1-Methylethylen und Butylen umfasst 1-Methylpropylen, 1,1-Dimethylethylen, 1,2-Dimethylethylen.
Die Definition für C₀-Alkylen bedeutet eine Bindung.

Unter dem Begriff "C₂₋₆-Alkenyl" (auch soweit sie Bestandteil anderer Reste sind) werden verzweigte und unverzweigte Alkenylgruppen mit 2 bis 6 Kohlenstoffatomen und unter dem Begriff "C₂₋₄-Alkenyl" verzweigte und unverzweigte Alkenylgruppen mit 2 bis 4 Kohlenstoffatomen verstanden, soweit sie mindestens eine Doppelbindung aufweisen. Bevorzugt sind Alkenylgruppen mit 2 bis 4 Kohlenstoffatomen. Beispielsweise werden hierfür genannt: Ethenyl oder Vinyl, Propenyl, Butenyl, Pentenyl, oder Hexenyl. Sofern nicht anders beschrieben, umfassen die Definitionen Propenyl, Butenyl, Pentenyl und Hexenyl alle denkbaren isomeren Formen der jeweiligen Reste. So umfasst beispielsweise Propenyl 1-Propenyl und 2-Propenyl, Butenyl umfasst 1-, 2- und 3-Butenyl, 1-Methyl-1-propenyl, 1-Methyl-2-propenyl etc..

Unter dem Begriff "C₂₋₆-Alkinyl" (auch soweit sie Bestandteil anderer Reste sind) werden verzweigte und unverzweigte Alkinylgruppen mit 2 bis 6 Kohlenstoffatomen und unter dem Begriff "C₂₋₄-Alkinyl" verzweigte und unverzweigte Alkinylgruppen mit 2 bis 4 Kohlenstoffatomen verstanden, soweit sie mindestens eine Dreifachbindung aufweisen. Beispielsweise werden hierfür genannt: Ethinyl, Propinyl, Butinyl, Pentinyl, oder Hexinyl. Sofern nicht anders beschrieben, umfassen die Definitionen Propinyl, Butinyl, Pentinyl und Hexinyl alle denkbaren isomeren Formen der jeweiligen Reste. So umfasst beispielsweise Propinyl 1-Propinyl und 2-Propinyl, Butinyl umfasst 1-, 2- und 3-Butinyl, 1-Methyl-1-propinyl, 1-Methyl-2-propinyl etc..

Unter dem Begriff "C₃₋₆-Cycloalkyl" (auch soweit sie Bestandteil anderer Reste sind) werden cyclische Alkylgruppen mit 3 bis 6 Kohlenstoffatomen und unter dem Begriff "C₅₋₆-Cycloalkyl" werden cyclische Alkylgruppen mit 5 bis 6 Kohlenstoffatomen verstanden. Beispielsweise werden hierfür genannt: Cyclopropyl, Cyclobutyl, Cyclopentyl oder Cyclohexyl. Soweit nicht anders beschrieben, können die cyclischen Alkylgruppen substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriff "C₅₋₆-Cycloalkenyl" (auch soweit sie Bestandteil anderer Reste sind) werden cyclische Alkenylgruppen mit 5 oder 6 Kohlenstoffatomen verstanden, die eine ungesättigte Bindung enthalten. Beispielsweise werden hierfür genannt: Cyclopentenyl oder Cyclohexenyl. Soweit nicht anders beschrieben, können die cyclischen Alkenylgruppen substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriff "Heterocyclyl" oder "Heterocyclus" werden, soweit in den Definitionen nicht anders beschrieben, stabile 5-, 6- oder 7-gliedrige monocyclische oder 8-, 9-, 10-oder 11-gliedrige bicyclische heterocyclische Ringsysteme verstanden, die in mindestens einem Ring kein aromatisches Ringsystem bilden und neben Kohlenstoffatomen ein bis vier Heteroatome tragen können, die ausgewählt sind aus der Gruppe bestehend aus Stickstoff, Sauerstoff und Schwefel. Dabei können sowohl Stickstoffatome als auch Schwefelatome optional oxidiert sein und Stickstoffatome können quaternisiert sein. Der heterocyclische Ring kann eine oder zwei Carbonyl-, Thiocarbonyl- oder Cyaniminogruppen benachbart zu einem Stickstoffatom enthalten. Die voranstehend genannten Heterocyclen können über ein Kohlenstoffatom oder über ein Stickstoffatom mit dem restlichen Molekül verknüpft sein. Soweit nicht anders beschrieben, können die Heterocyclen substituiert sein mit einem oder mehreren Resten ausgewählt aus der Gruppe bestehend aus:
(a) OH, NO₂, CN, OCF₃, OCHF₂, OCH₂F, NH₂,
(b) Halogen, vorzugsweise Fluor oder Chlor,
(c) C₁₋₆-Alkyl, vorzugsweise C₁₋₃-Alkyl, besonders bevorzugt Ethyl, Methyl, *iso-*Propyl oder *tert*-Butyl,
(d) -SO₂-O-C₁₋₃-Alkyl, vorzugsweise -O-Methyl,
(e) -O-C₁₋₃-Alkyl, vorzugsweise-O-Methyl oder-O-Ethyl,
(f) COOH, COO-C₁₋₃-Alkyl, vorzugsweise CO-O-Methyl oder CO-O-Ethyl,
wobei die Reste gleich oder verschieden sein können.
Als Beispiele seien folgende Verbindungen genannt, sollen jedoch nicht auf diese beschränkt sein: Azetidin, Oxetan, Thietan, Thietandioxid, Tetrahydrofuran, Dihydrofuran, Dioxalan, Imidazolidin, Imidazolin, Imidazolidinon, Dihydroimidazolon, Oxazolin, Oxazolidin, Oxazolidinon, Pyrrolidinon, Dihydropyrazol, Pyrrolidin, Pyrrolin, Morpholin, Tetrahydropyridin, Dihydropyran, Tetrahydropyran, Dioxan, Piperazin, Piperidin, Piperazinon, Piperidinon, Pyran, Thiomorpholin-S-oxid, Thiomorpholin-S-dioxid, Thiomorpholin, Dihydrooxazin, Morpholindion, Morpholinthion, Perhydrothiazindioxid, ε-caprolactam, Oxazepanon, Diazepanon, Thiazepanon, Perhydroazepin, Dihydrochinazolinon, Dihydroindol, Dihydroisoindol, Benzoxazolon, Benzimidazolon, Chromanon, Tetrahydrochinolin, Tetrahydrobenzoxazol, Tetrahydrobenzisoxazol, Tetrahydrobenzthiophen, Tetrahydrothieno-pyridin, Tetrahydrobenzofuran, Tetrahydrooxazolopyridin, Tetrahydro-isoxazolopyridin.

### Bevorzugt im Sinne dieser Erfindung seien folgende Heterocyclen:

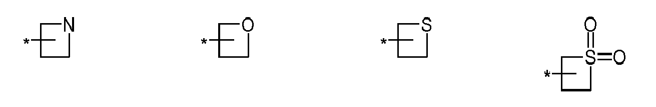

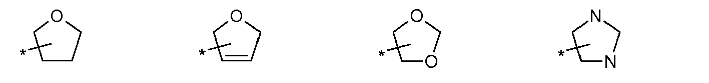

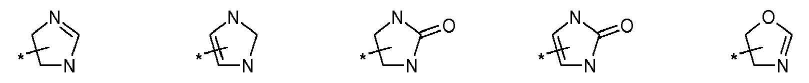

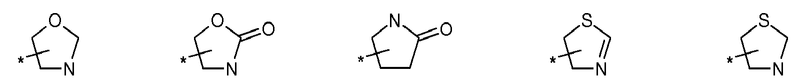

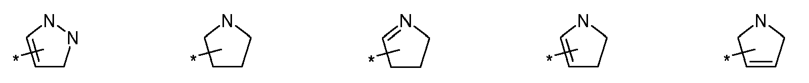

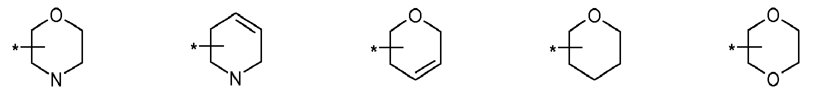

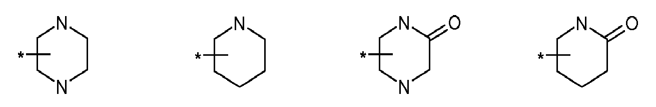

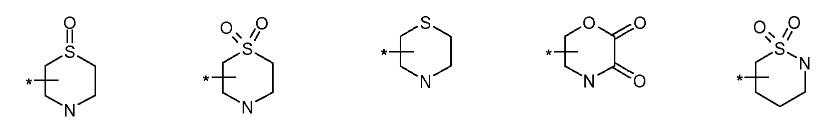

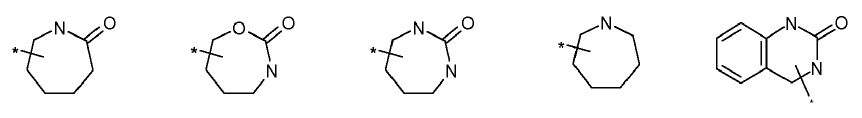

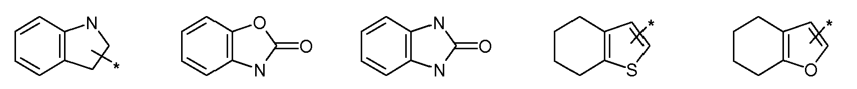

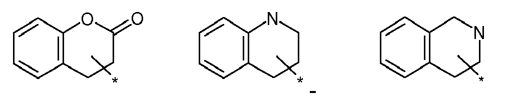

Unter dem Begriff "Aryl" (auch soweit sie Bestandteil anderer Reste sind) werden monocyclische aromatische Ringsysteme mit 6 Kohlenstoffatomen oder bicyclische aromatische Ringsysteme mit 10 Kohlenstoffatomen verstanden. Beispielsweise werden hierfür Phenyl, 1-Naphthyl oder 2-Naphthyl genannt; bevorzugter Arylrest ist Phenyl. Soweit nicht anders beschrieben, können die Aromaten substituiert sein mit einem oder mehreren Resten ausgewählt aus der Gruppe bestehend aus:
(a) OH, NO₂, CN, OCF₃, OCHF₂, OCH₂F, NH₂,
(b) Halogen, vorzugsweise Fluor oder Chlor,
(c) C₁₋₆-Alkyl, vorzugsweise C₁₋₃-Alkyl, besonders bevorzugt Ethyl, Methyl, *iso-*Propyl oder *tert*-Butyl,
(d) -SO₂-O-C₁₋₃-Alkyl, vorzugsweise -O-Methyl,
(e) -O-C₁₋₃-Alkyl, vorzugsweise -O-Methyl oder-O-Ethyl,
(f) COOH, CO-O-C₁₋₃-Alkyl, vorzugsweise CO-O-Methyl oder CO-O-Ethyl,
wobei die Reste gleich oder verschieden sein können.

Unter dem Begriff "Heteroaryl" werden stabile fünf- oder sechsgliedrige heterocyclische Aromaten oder 8- bis 10-gliedrige bicyclische Heteroarylringe verstanden, die in jedem Ring ein, zwei oder drei Heteroatome, ausgewählt aus der Gruppe Sauerstoff, Schwefel und Stickstoff, enthalten können und zusätzlich so viele konjugierte Doppelbindungen enthalten, dass ein aromatisches System gebildet wird. Als Beispiele für fünf- oder sechsgliedrige heterocyclische Aromaten werden genannt, sollen aber nicht auf diese beschränkt sein: Furan, Pyrrol, Thiophen, Pyrazol, Imidazol, Oxazol, Thiazol, Isothiazol, Isoxazol, Oxadiazol, Triazol, Tetrazol, Furazan, Thiadiazol, Pyridin, Pyrimidin, Pyrazin, Pyridazin, Triazin.

Bevorzugt im Sinne dieser Erfindung seien folgende fünfgliedrige heterocyclische Aromaten:

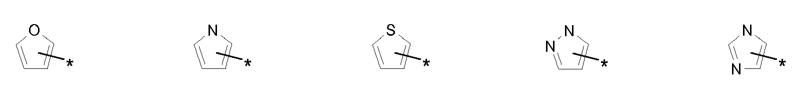

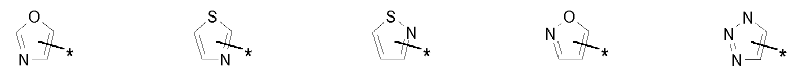

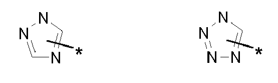

Bevorzugt im Sinne dieser Erfindung seien folgende sechsgliedrige heterocyclische Aromaten:

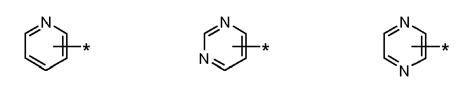

Als Beispiele für 9- oder 10-gliedrige bicyclische Heteroarylringe werden genannt, sollen aber nicht auf diese beschränkt sein: Indol, Isoindol, Indazol, Indolizin, Benzofuran, Benzthiophen, Benzimidazol, Benzoxazol, Benzthiazol, Benztriazol, Benzisoxazol, Benzisothiazol, Chinolin, Isochinolin, Cinnolin, Phtalazin, Chinoxalin, Chinazolin, Pyridopyrimidin, Pyridopyrazin, Pyridopyridazin, Pyrimidopyrimidin, Pteridin, Purin, Chinolizin, Benzoxazolcarbonitril, Chinolin, Isochinolin, Chinolizin, Pteridin, Purin, Chinolizin, Benzoxazol-carbonitril.

### Bevorzugt im Sinne dieser Erfindung seien folgende bicyclische Heteroarylringe:

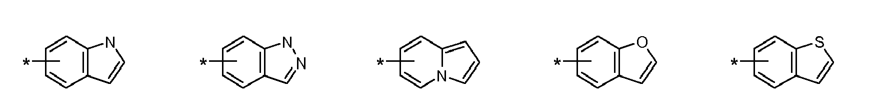

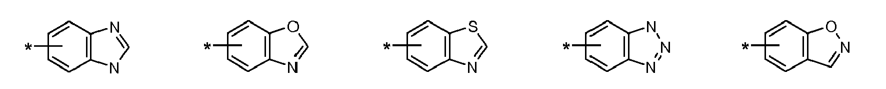

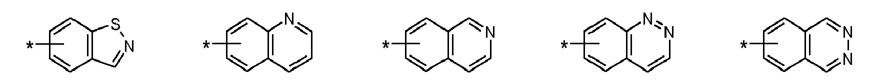

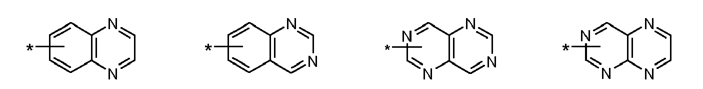

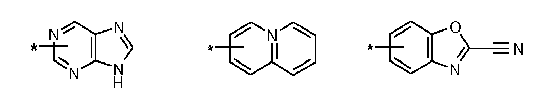

Soweit nicht anders beschrieben, können die voranstehend genannten Heteroaryle substituiert sein mit einem oder mehreren Resten ausgewählt aus der Gruppe bestehend aus:
(a) OH, NO₂, CN, OCF₃, OCHF₂, OCH₂F, NH₂,
(b) Halogen, vorzugsweise Fluor oder Chlor,
(c) C₁₋₆-Alkyl, vorzugsweise C₁₋₃-Alkyl, besonders bevorzugt Ethyl, Methyl, iso-Propyl oder *tert*-Butyl,
(d) -SO₂-O-C₁₋₃-Alkyl, vorzugsweise -O-Methyl,
(e) -O-C₁₋₃-Alkyl, vorzugsweise -O-Methyl oder-O-Ethyl,
(f) COOH, CO-O-C₁₋₃-Alkyl, vorzugsweise CO-O-Methyl oder CO-O-Ethyl,
wobei die Reste gleich oder verschieden sein können.

Bicyclische Heteroarylringe können dabei vorzugsweise im Phenylrest substituiert sein.

Unter dem Begriff "Halogen" werden Fluor-, Chlor-, Brom- oder lodatome verstanden.

Verbindungen der allgemeinen Formel **I** können Säuregruppen besitzen, hauptsächlich Carboxylgruppen, und/oder basische Gruppen wie z.B. Aminofunktionen. Verbindungen der allgemeinen Formel **I** können deshalb als innere Salze, als Salze mit pharmazeutisch verwendbaren anorganischen Säuren wie beispielsweise Bromwasserstoffsäure, Phosphorsäure, Salpetersäure, Salzsäure, Schwefelsäure, Methansulfonsäure, Ethansulfonsäure, Benzolsulfonsäure, *p*-Toluolsulfonsäure oder organischen Säuren wie beispielsweise Äpfelsäure, Bernsteinsäure, Essigsäure, Fumarsäure, Maleinsäure, Mandelsäure, Milchsäure, Weinsäure, Zitronensäure oder als Salze mit pharmazeutisch verwendbaren Basen wie Alkali- oder Erdalkalimetallhydroxiden, beispielsweise Natriumhydroxid oder Kaliumhydroxid, oder Carbonaten, Ammoniak, Zink- oder Ammoniumhydroxiden oder organischen Aminen wie z.B. Diethylamin, Triethylamin, Ethanolamin, Diethanolamin, Triethanolamin, Cyclohexylamin, Dicyclohexylamin u.a. vorliegen.

Die erfindungsgemäßen Verbindungen können als Racemate vorliegen, sofern sie nur ein Chiralitätselement besitzen, sie können aber auch als reine Enantiomere, d.h. in (*R*)- oder (*S*)-Form gewonnen werden.

Die Anmeldung umfasst jedoch auch die einzelnen diastereomeren Antipodenpaare oder deren Gemische, die dann vorliegen, wenn mehr als ein Chiralitätselement in den Verbindungen der allgemeinen Formel **I** vorhanden ist, sowie die einzelnen optisch aktiven Enatiomeren, aus denen sich die erwähnten Racemate zusammensetzen.

Gegenstand der Erfindung sind die jeweiligen Verbindungen gegebenenfalls in Form der einzelnen optischen Isomeren, Mischungen der einzelnen Enantiomeren oder Racemate, in Form der Tautomere sowie in Form der freien Basen oder der entsprechenden Säureadditionssalze mit pharmakologisch unbedenklichen.

Sogenannte Prodrugs von Verbindungen der allgemeinen Formel **I** sind ebenfalls Gegenstand dieser Erfindung. Als Prodrug wird jedes Molekül bezeichnet, das nach Applikation an Säugetieren das aktive Prinzip der allgemeinen Formel **I** in-vivo freisetzt. Das Prodrug kann per se keine oder nur geringe pharmakologische Aktivität haben, es setzt allerdings nach Verabreichung in-vivo das aktive Prinzip der allgemeinen Formel **I** frei und dieses weist die beschriebene Aktivität auf. Prodrugs für Verbindungen der allgemeinen Formel **I** können hergestellt werden, indem geeignete funktionelle Gruppen in der Verbindung der allgemeinen Formel **I** so modifiziert werden, wie es einem Fachmann in diesem Gebiet bekannt ist. (H. Bundgaard (Editor), Design of Prodrugs. (1986), Elsevier)

Diese Erfindung umfasst auch jene Metaboliten, die sich aus der Verbindungen der allgemeinen Formel **I** ableiten. Unter Metaboliten versteht man in diesem Zusammenhang solche Verbindungen, die nach Applikation in-vivo aus der Verbindung der allgemeinen Formel **I** entstehen. Als Beispiel für Metaboliten werden genannt:
- Methylgruppen der Verbindung der allgemeinen Formel **I** können in die entsprechenden Hydroxymethyl-Gruppen überführt werden. (-CH₃ -> -CH₂OH)
- Alkoxy-Gruppen der Verbindung der allgemeinen Formel **I** können in die entsprechenden Hydroxyl-Gruppen überführt werden. (-OR -> -OH)
- Sekundäre Amine der Verbindung der allgemeinen Formel **I** können in die entsprechenden primären Amine überführt werden. (-NR₁R₂ -> -NHR₁ oder -NHR₂)
- Stickstoff-Atome der Verbindung der allgemeinen Formel **I** können in die entsprechenden Stickstoff-Oxide überführt werden. (=N- -> =N⁺-(O⁻)-)

### HERSTELLVERFAHREN

Gegenstand der Erfindung ist weiterhin ein Verfahren zur Herstellung der Verbindungen der allgemeinen Formel **I**, wobei die Substituenten die voranstehend genannten Bedeutungen haben.

Einige Methoden zur Herstellung der erfindungsgemäßen Verbindungen der allgemeinen Formel **I** 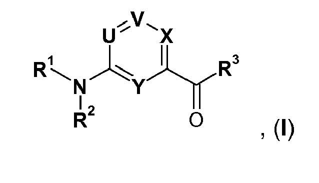 in der **U, V, X, Y, R¹, R²** und **R³** wie voranstehend erwähnt definiert sind, sind in den folgenden Syntheseschemata und Beispielen dargestellt.

In einigen Fällen kann die Reihenfolge bei der Durchführung der Reaktionsschemata variiert werden, um die Reaktionen zu vereinfachen oder um unerwünschte Nebenprodukte zu verhindern. Die nachfolgenden Beispiele sind genannt, um die Erfindung verständlich zu machen. Die Beispiele sollen der Anschaulichkeit der Erfindung dienen und sollen die Erfindung in keinster Weise einschränken.

In einigen Fällen kann das Endprodukt weiter derivatisiert werden, z.B. durch Manipulation der Substituenten. Diese Manipulationen können die dem Fachmann allgemein bekannten sein wie Oxidation, Reduktion, Alkylierung, Acylierung und Hydrolyse, müssen allerdings nicht auf diese beschränkt sein.

Ausgangsverbindungen werden gemäß Verfahren hergestellt, die entweder in dem Fachgebiet bekannt sind oder hierin beschrieben werden. Vor Durchführung der Reaktion können in den Verbindungen entsprechende funktionelle Gruppen durch übliche Schutzreste geschützt werden. Diese Schutzgruppen können auf einer geeigneten Stufe innerhalb der Reaktionssequenz nach dem Fachmann bekannten Methoden wieder abgespalten werden (P.G.M. Wuts, T.W. Greene "Greene's protective groups in organic synthesis", 4th ed., Wiley Interscience).

Die erfindungsgemäßen Verbindungen können gemäß den dargestellten Schemata und spezifischen Beispielen oder entsprechenden Modifikationen davon hergestellt werden, indem man bekannte und/oder verfügbare Startmaterialien, Reagenzien und herkömmliche Syntheseverfahren verwendet. Von diesen Reaktionen können auch Abwandlungen eingesetzt werden, die einem Fachmann bekannt sind, hier aber nicht detailliert beschrieben sind.

Die folgenden Verfahren zur Herstellung der erfindungsgemäßen Verbindungen der allgemeinen Formel **I** und ihrer Vorstufen und ihrer Vorstufen haben sich besonders bewährt:
Ausgangsverbindungen sind kommerziell verfügbar oder werden gemäß Verfahren hergestellt, die in der Literatur beschrieben sind, in dem Fachgebiet dem Fachmann bekannt sind oder wie sie hierin beschrieben sind. Vor Durchführung der Reaktion können in den Verbindungen entsprechende funktionelle Gruppen durch übliche Schutzreste geschützt werden. Diese Schutzgruppen können auf einer geeigneten Stufe innerhalb der Reaktionssequenz nach für den Fachmann geläufigen Methoden wieder abgespalten werden.
Bei den nachstehend beschriebenen Umsetzungen können gegebenenfalls vorhandene reaktive Gruppen wie Hydroxy-, Carboxy-, Amino-, Alkylamino-, Amid- oder Iminogruppen während der Umsetzung durch übliche Schutzgruppen geschützt werden, welche nach der Umsetzung wieder abgespalten werden. Beispielsweise kommt
   - als Schutzrest für eine Hydroxygruppe die Methoxy-, Benzyloxy-,Trimethylsilyl-, Acetyl-, Benzoyl-, tert.-Butyl-, Trityl-, Benzyl- oder Tetrahydropyranylgruppe,
   - als Schutzreste für eine Carboxylgruppe die Trimethylsilyl-, Methyl-,Ethyl-, tert.-Butyl-, Benzyl- oder Tetrahydropyranylgruppe, und
   - als Schutzreste für eine Amidgruppe die N-Methoxymethyl- (MOM), N-Benzyloxymethyl- (BOM), N-(Trimethylsilyl)ethoxymethyl (SEM), N-*tert*-Butyldimethylsiloxymethyl, N-tert-Butyldimethylsilyl- (TBDMS), N-Triisopropylsilyl- (TIPS), N-Benzyl-, N-4-Methoxybenzyl- (PMB), N-Triphenylmethyl- (Trt), N-tert-Butoxycarbonyl-(BOC), N-Benzyloxycarbonyl- (Cbz), N-Trimethylsilylethylsulfonyl- (SES)
   - als Schutzrest für eine Amino-, Alkylamino- oder Iminogruppe die Acetyl-, Trifluoracetyl-, Benzoyl-, Ethoxycarbonyl-, tert.-Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe, in Betracht.

Weitere Schutzgruppen und deren Abspaltung sind in T.W. Greene, P.G.M. Wuts, "Protective Groups in Organic Synthesis", Wiley, 2006 beschrieben.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z. B. in Gegenwart von lodtrimethylsilan, bei Temperaturen zwischen 0°C und 100°C, vorzugsweise bei Temperaturen zwischen 10°C und 50°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0°C und 50°C, vorzugsweise jedoch bei Raumtemperatur und bei einem Wasserstoffdruck von 1 bis 7 bar,vorzugsweise jedoch von 1 bis 5 bar.

Die Abspaltung einer Methoxybenzylgruppe kann auch in Gegenwart eines Oxidationsmittels wie Cer(IV)ammoniumnitrat in einem Lösungsmittel wie Methylenchlorid, Acetonitril oder Acetonitril/Wasser bei Temperaturen zwischen 0°C und 50°C, vorzugsweise jedoch bei Raumtemperatur, erfolgen.

Die Abspaltung einer Methoxygruppe erfolgt zweckmäßigerweise in Gegenwart von Bortribromid in einem Lösungsmittel wie Methylenchlorid bei Temperaturen zwischen -35 und -25°C.

Die Abspaltung eines 2,4-Dimethoxybenzylrestes erfolgt vorzugsweise in Trifluoressigsäure in Gegenwart von Anisol.

Die Abspaltung eines tert.-Butyl- oder tert.-Butoxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan oder Ether.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20°C und 50°C.

Die Abspaltung eines Methoxymethyl-Rests kann in Gegenwart einer Säure wie konzentrierter Salzsäure in einem Lösungsmittel wie Dimethoxyethan durchgeführt werden. Alternativ kann eine Säure wie Trifluoressigsäure auch ohne Lösungsmittel eingesetzt werden.

Die Abspaltung eines N-(Trimethylsilyl)ethoxymethyl-Rests kann in Gegenwart von TBAF und 1,3-Dimethyl-3,4,5,6-tetrahydro-2(1H)-Pyrimidon durchgeführt werden. Alternativ kann die SEM-Schutzgruppe auch mit einer Säure wie Chlorwasserstoff in einem organischen Lösungsmittel wie Dioxan oder Ethanol durchgeführt werden.

Die Abspaltung eines Allyloxycarbonylrestes erfolgt durch Behandlung mit einer katalytischen Menge Tetrakis-(triphenyl-phosphin)-palladium(0) vorzugsweise in einem Lösungsmittel wie Tetrahydrofuran und vorzugsweise in Gegenwart eines Überschusses von einer Base wie Morpholin bei Temperaturen zwischen 0°C und 100°C, vorzugsweise bei Raumtemperatur und unter Inertgas, oder durch Behandlung mit einer katalytischen Menge von Tris-(triphenylphosphin)-rhodium(I)chlorid in einem Lösungsmittel wie wässrigem Ethanol und gegebenenfalls in Gegenwart einer Base wie 1,4-Diazabicyclo[2.2.2]octan bei Temperaturen zwischen 20°C und 70°C.

Die folgenden Verfahren zur Herstellung der erfindungsgemäßen Verbindungen der allgemeinen Formel **I** und ihrer Vorstufen haben sich besonders bewährt:
Die Herstellung einer Endverbindung der allgemeinen Formel **I**, in der **U, V, X, Y, R¹, R²** und **R³** wie voranstehend erwähnt definiert sind, kann durch Reaktion einer Verbindung der allgemeinen Formel (1-1) mit einer elektronenarmen Verbindung der allgemeinen Formel (1-2), die eine Austrittsgruppe LG besitzt, erfolgen. Als Austrittsgruppe LG können Halogenide, bevorzugt Chloride und Bromide, -SO₂CH₃, -OSO₂CH₃, -OSO₂C₆H₄-CH₃ oder
-S-CH₃ (-S-CH₃ erfordert die weitere Umsetzung mit einem organischen Peroxid um in die eigentliche Austrittsgruppe überführt werden zu können) etc. fungieren, müssen aber nicht auf diese beschränkt sein. Ganz besonders bevorzugt ist die Verwendung von Chloriden.

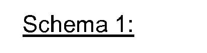

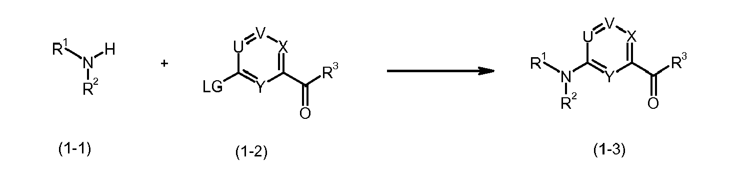

Die Reaktion kann über eine nucleophile aromatische Substitution in einem inerten Lösungsmittel unter Verwendung einer Hilfsbase in einem Temperaturbereich von 0°C bis zum Rückfluss des Lösungsmittels erfolgen. Die Reaktion wird in einem geeigneten, inerten Lösungsmittel, wie Tetrahydrofuran, Toluol, Xylol, Dialkylformamid (besonders bevorzugt Dimethylformamid), cyclische Amide (besonders bevorzugt N-Methyl-pyrrolidon), 1,4-Dioxan, Acetonitril oder in inerten Lösungsmittelgemischen durchgeführt. Als geeignete Hilfsbasen können tertiäre Amine wie Triethylamin oder Ethyldiisopropylamin, Alkalimetal Carbonate wie Kaliumcarbonat oder Natriumcarbonat, Natriumhydrid (NaH) oder Lithium diisopropylamid (LDA) verwendet werden. Dabei muss das verwendete inerte Lösungsmittel kompatibel sein mit der verwendeten Base. Bevorzugt wird die Reaktion in Dimethylformamid, bei Temperaturen zwischen Raumtemperatur und Rückfluss des Lösungsmittels, in Gegenwart einer tertiären Aminbase durchgeführt.

Alternativ können die in Schema 1 dargestellten Strukturen der allgemeinen Form (1-3), in der **U, V, X, Y, R¹, R²** und **R³** wie voranstehend erwähnt definiert sind, über Übergangsmetall-katalysierte Reaktionen synthetisiert werden. Dabei kann eine Verbindung der allgemeinen Formel (1-1) mit einer Verbindung der allgemeinen Formel (1-2), die eine Austrittsgruppe LG besitzt, in einem inerten Lösungsmittel bei Anwesenheit eines Katalysators und einer Hilfsbase reagieren. Für den Katalysator kann zusätzlich ein geeigneter Ligand verwendet werden. Als Austrittsgruppe LG können Chloride, Bromide, Iodide, Trifluoracetate, Trifluormethansulfonate, Methansulfonate und Toluolsulfonate fungieren, müssen aber nicht auf diese beschränkt sein. Als inerte Lösungsmittel können Xylol, Tetrahydrofuran, Dimethylformamid, Dimethoxyethan, Toluol, Benzol, 1,4-Dioxan, Acetonitril oder Lösungsmittelgemische verwendet werden. Bevorzugtes Lösungsmittel ist Xylol. Geeignete Basen sind besonders Amin-Basen wie z.B. Triethylamin oder Diisopropylethylamin oder auch anorganische Basen wie Cäsiumcarbonat, Cäsiumacetat, Kaliumcarbonat, Natriumcarbonat oder Kaliumphosphat. Bevorzugte Reaktionstemperaturen sind von RT bis Rückfluss des Lösungsmittels bei Normaldruck. Typische Katalysatoren sind z.B. Übergangsmetall-Katalysatoren, wie z.B. Palladium-Katalysatoren der Art Tris(dibenzylidenaceton)-dipalladium(0), Tetrakis-(triphenylphosphin)-palladium(0), Palladium-(II)-acetat, Pd(PPh₃)₂Cl₂, Pd(CH₃CN)₂Cl₂, Pd(dppf)Cl₂ oder Palladium(II)-chlorid. Typische Liganden sind z.B. Triphenylphosphin, Triphenylarsen, BINAP, XPhos, XantPhos, oder 2-(Di-*tert*-butylphosphino)biphenyl.

Verbindungen der allgemeinen Formel (2-4), in der **U, V, X, Y, R¹, R²** und **R³** wie voranstehend erwähnt definiert sind, können wie in Schema 2 gezeigt, dargestellt werden.

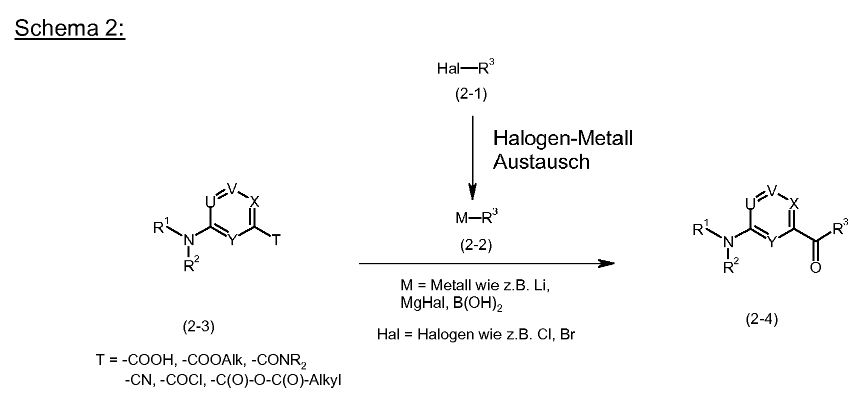

Die Reaktion geht von einer Verbindung der allgemeinen Form (2-1) aus, bei der Hal für ein Halogenatom, bevorzugt Chlor, Brom oder Iod steht. Die Grignard oder lithiierte Verbindung der allgemeinen Formel (2-2) kann aus der entsprechend halogenierten Verbindung der allgemeinen Form (2-1) entweder durch einen sogenannten Halogen-Metall Austausch oder durch Insertion des Metalls in eine Halogen-Kohlenstoff-Bindung erfolgen. Um die entsprechende lithiierte Verbindung der allgemeinen Formel (2-2) zu synthetisieren kann der Halogen-Metall-Austausch z.B. mit einer Organolithium-Verbindung wie z.B. n-, sec- oder tert.-Butyllithium erfolgen. Die entsprechenden Magnesium-Verbindungen (Grignard-Verbindungen) können ebenfalls duch einen Halogen-Metall-Austausch mit einem entsprechenden Grignard-Reagenz wie z.B. Isopropyl- oder sec-Butyl-Magnesium-Bromid oder Isopropyl- oder sec-Butyl-Magnesium-Chlorid oder Diisopropyl- oder Di-sec-Butylmagnesium mit oder in Gegenwart eines Salzes wie z.B. Lithiumchlorid, das den Metallierungs-Prozess beschleunigen kann, erhalten werden. Die entsprechende transmetallierende Organo-Magnesium-Verbinung kann ebenso in-situ aus entsprechenden Vorstufen synthetisiert werden. (siehe z.B. Angew. Chem. 2004, 116, 3396-3399 und Angew. Chem. 2006, 118, 165-169 und darin enthaltene Referenzen). Darüber hinaus können auch -ate Komplexe der Organo-Magnesium-Verbindungen verwendet werden, die aus der Kombination von z.B. Butylmagnesiumchlorid oder - Bromid oder Isopropyl-Magnesiumchlorid oder -Bromid und Butyllithium resultieren. (siehe Angew. Chem. 2000, 112, 2594-2596 und Tetrahedron Lett. 2001, 42, 4841-4844 und darin enthaltene Referenzen). Der Halogen-Metall-Austausch wird bevorzugt zwischen -100°C und 40°C durchgeführt, ganz besonders bevorzugt ist ein Temperaturbereich von -80°C und 10°C in einem inerten Lösungsmittel, bevorzugt sind Alkylether (ganz besonders bevorzugt Diethylether), cyclische Ether (ganz besonders bevorzugt 1,4-Dioxan oder Tetrahydrofuran), Toluol, Hexan oder Lösungsmittelgemische davon. Die so erhaltenen Magnesium- oder Lithium-Organo-Verbindungen können optional transmetalliert werden mit Metallsalzen wie z.B. Certrichlorid, Zinkchlorid oder Zinkbromid, Indiumchlorid oder Indiumbromid um alternative Organometall-Verbindungen der allgemeinen Form (2-2) zu synthetisieren, die sich ebenfalls für die beschriebene Reaktion eignen. Alternativ kann die Organometall-Verbindung (2-2) ebenso durch Insertion eines Metalls in eine Kohlenstoff-Halogen-Bindungen erfolgen. Lithium oder Magnesium sind geeignete elementare Metalle für diese Transformation. Die Insertions-Reaktion wird bevorzugt zwischen -80°C und 100°C durchgeführt, ganz besonders bevorzugt ist ein Temperaturbereich von -70°C bis 40°C in einem inerten Lösungsmittel, bevorzugt sind Alkylether (ganz besonders bevorzugt Diethylether), cylische Ether (ganz besonders bevorzugt 1,4-Dioxan oder Tetrahydrofuran), Toluol, Hexan oder Lösungsmittelgemische davon. In Fällen, in denen keine spontane Reaktion stattfindet, ist gegebenenfalls eine Aktivierung des Metalls mit z.B. 1,2-Dibromethan, Iod, Trimethylsilylchlorid, Essigsäure, Chlorwasserstoff oder Ultraschall erforderlich. Die Umsetzung der Organometall-Verbindung der allgemeinen Formel (2-2) mit einer Verbindung (2-3) wird bevorzugt in einem Temperaturbereich von -100°C bis 100°C durchgeführt, besonders bevorzugt ist ein Temperaturbereich von -80°C bis 50°C. Die Reaktion wird in einem inerten Lösungsmittel durchgeführt, wie z.B. bevorzugt sind Alkylether (ganz besonders bevorzugt Diethylether, Dimethoxyethan), cyclische Ether (ganz besonders bevorzugt 1,4-Dioxan oder Tetrahydrofuran), aromatische Kohlenwasserstoffe (ganz besonders bevorzugt sind Toluol oder Benzol), Hexan oder Lösungsmittelgemische davon. Alle Reaktionen können an Luft durchgeführt werden, allerdings ist die Durchführung in einer Schutzgas-Atmosphäre wie Argon oder Stickstoff bevorzugt. Es kann sich als vorteilhaft erweisen, wenn funktionelle Gruppe in Verbindung (2-3) temporär geschützt werden. Die Lithium-substituierte oder Magnesium-substituierte Verbindung der allgemeinen Formel (2-2) kann mit einer Verbindung der allgemeinen Formel (2-3) in gewünschter Weise reagieren, die eine Carboxyl-Gruppe oder Derivate davon wie Ester, Nitrile, Carbonsäurechloride oder Amide, wie z.B. Weinreb-Amide enthalten. Diese Reaktionen können häufig ohne zusätzlichen Übergangsmetall-Katalysator oder Ummetallierung auf ein anderes Metall wie z.B. Cer, Indium oder Zink durchgeführt werden. In einigen Fällen können sich die beiden genannten Abwandlungen aber auch als vorteilhaft erweisen. Aromatische Boronsäuren, davon abgeleitete Ester, Dialkylarylborane oder Aryltrifluorborate können mit Säurechloriden oder Carbonsäuren in Gegenwart eines Übergangsmetalls, wie z.B. Palladium als Katalysator zu den entsprechenden Ketonen umgesetzt werden (V. Polackova, St. Toma, I. Augustinova, Iveta; Tetrahedron; 2006; 62; 50; 11675-11678 und darin zitierte Refrenzen und R. Kakino, H. Narahashi, I. Shimizu, A. Yamamoto, Bull. Chem. Soc. Jpn., 2002, 75, 1333-1345). Die entsprechenden Bor-substituierten Verbindung, wie z.B. Boronsäuren, Dialkylarylborane oder Boronsäureester lassen sich aus der metallierten Spezies durch Reaktion mit einem Bor-Elektrophil wie z.B. Borsäureester oder Derivate davon synthetisieren. Bor-substituierte Verbindungen können darüber hinaus aus den halogenierten bzw. pseudohalogenierten Vorläufer-Molekülen mittels eines Übergangsmetall-Katalysators, bevorzugt Palladium, und einer Bor- bzw. Borolan-Verbindung synthetisiert werden. (Tetrahedron Lett. 2003, 4895-4898 und darin zitierte Referenzen).

Die Metallierung und/oder Kupplungsreaktion kann auch in Microreaktoren und/oder im Micromixer durchgeführt werden. Die Additionsreaktionen können ohne weitere Zusätze durchgeführt werden oder im Falle von schlecht reagierenen Reaktionspartnern können auch Promotoren wie z.B. BF₃*OEt₂ zugesetzt werden (siehe M.Schlosser, Organometallics in Synthesis, John Wiley & Sons, Chichester/ New York/ Brisbane/Toronto/ Singapor, 1994)

Die halogenierten Verbindungen der allgemeinen Formel (2-1) sind entweder kommerziell verfügbar oder können nach Verfahren synthetisiert werden, die im Fachgebiet der organischen Chemie bekannt sind oder in der Fachliteratur beschrieben sind (siehe z.B. J. March, Advanced Organic Reactions, Reactions Mechanism, and Structure, 4th Edition, John Wiley & Sons, Chichester/New York/Brisbane/Toronto/Singapore, 1992 und darin zitierte Literatur). Die Verwendung von Übergangsmetallen und Organometall-Verbindungen für die Synthese ist detailiert in Monographien beschrieben. (siehe z.B. L. Brandsma, S.F. Vasilevsky, H.D. Verkruijsse, Application of Transition Metals Catalysts in Organic Synthesis, Springer-Verlag, Berlin/Heidelberg, 1999; M. Schlosser, Organometallics in Synthesis, John Wiley & Sons, Chichester/ New York/ Brisbane/ Toronto/ Singapor, 1994, P.J. Stang, F. Diederich, Metal-Catalyzed Cross-Coupling Reactions, Wiley-VCH, Weinheim, 1997 and darin enthaltene Referenzen.)

Eine Synthese für Verbindungen der allgemeinen Formel (3-4), in der **U, V, X, Y** und **R³** wie voranstehend erwähnt definiert sind, ist in Schema 3 dargestellt.

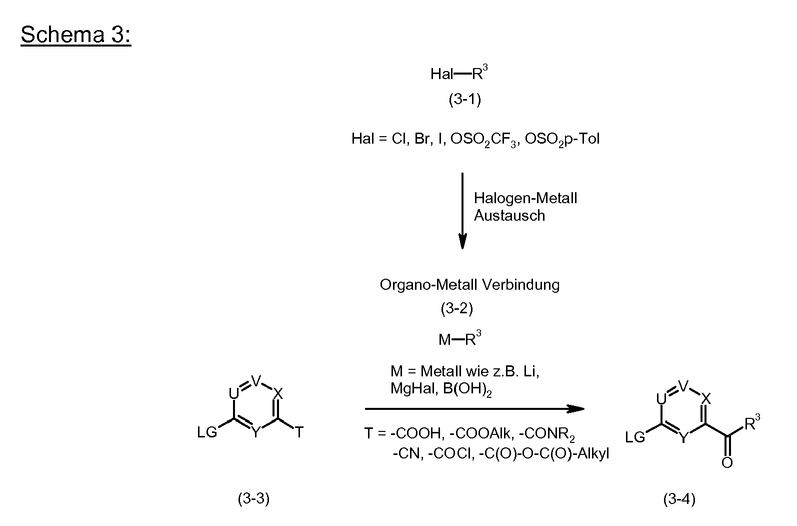

Ausgehend von einer halogenierten Verbindung (besonders bevorzugt sind die Chloride, Bromide und Iodide) der allgemeinen Formel (3-1) kann durch eine Halogen-Metall-Austauschreaktion z.B. mit Butyllithium, Isopropylmagnesium Halogenid oder Diisopropylmagnesium oder auch durch Insertion eines elementaren Metalls in die Halogen-Kohlenstoff-Bindung die entsprechende Lithium- oder Magnesium-substituierte Verbindung synthetisiert werden. Die entsprechenden Bor-substituierten Verbindungen, wie z.B. Boronsäure, Dialkylarylboran oder Boronsäureester lassen sich aus der metallierten Spezies durch Reaktion mit einem Bor-Elektrophil wie z.B. Borsäureester oder Derivate davon synthetisieren. Bor-substituierte Verbindungen können darüber hinaus aus den halogenierten bzw. pseudohalogenierten Vorläufer-Molekülen mittels eines Übergangsmetall-Katalysators, bevorzugt Palladium, und einer Bor- bzw. Borolan-Verbindung synthetisiert werden. (Tetrahedron Lett. 2003, 4895-4898 und darin zitierte Referenzen)

Die Lithium-substituierte oder Magnesium-substituierte Verbindung der allgemeinen Formel (3-2) kann an eine Verbindung der allgemeinen Formel (3-3) addieren, die eine Carboxyl-Gruppe oder Derivate davon wie Ester, Nitrile, Carbonsäurechloride oder Amide, wie z.B Weinreb-Amide enthalten. Diese Reaktionen können häufig ohne zusätzlichen Übergangsmetall-Katalysator oder Ummetallierung auf ein anderes Metall wie z.B. Cer, Indium oder Zink durchgeführt werden. In einigen Fällen können sich die beiden genannten Abwandlungen aber auch als vorteilhaft erweisen. Aromatische Boronsäuren, davon abgeleitete Ester, Dialkylarylborane oder Aryltrifluorborate können mit Säurechloriden oder Carbonsäuren in Gegenwart eines Übergangsmetalls, wie z.B. Palladium als Katalysator zu den entsprechenden Ketonen umgesetzt werden (V. Polackova, St. Toma, I. Augustinova, Iveta; Tetrahedron; 2006; 62; 50; 11675-11678 und darin zitierte Refrenzen und R. Kakino, H. Narahashi, I. Shimizu, A. Yamamoto, Bull. Chem. Soc. Jpn., 2002, 75, 1333-1345)

Verbindungen der allgemeinen Formel (4-3), in der **U, V, X, Y** und **R³** wie voranstehend erwähnt definiert sind, können wie in Schema 4 gezeigt, dargestellt werden.

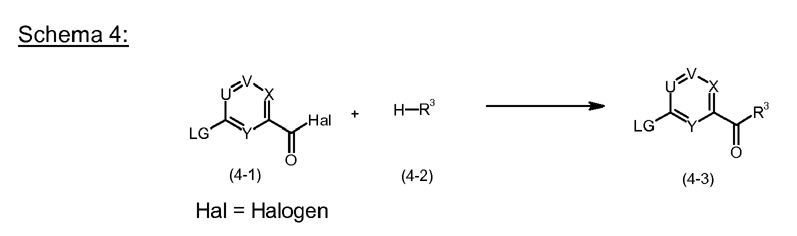

Eine Verbindung der allgemeinen Formel (4-1), die eine Austrittsgruppe LG und eine Säurehalogenid-Gruppe besitzt, kann mit einer aromatischen Verbindung der allgemeinen Formel (4-2) unter Friedel-Crafts Acylierungs Bedingungen bzw. Variationen davon, zur Reaktion gebracht werden. Friedel-Crafts-Reaktionen werden in Gegenwart eines Katalysators durchgeführt, der entweder in katalytischen oder stöchiometrischen Mengen eingesetzt wird. Als Katalysator eignen sich vor allem AlCl₃, FeCl₃, Iod, Eisen, ZnCl₂, Schwefelsäure oder Trifluormethansulfonsäure. Anstelle des Säurehalogenids kann auch die entsprechende Carbonsäure, Anhydrid, Ester oder Nitril verwendet werden. Die Reaktion wird bevorzugt in halogenierten Kohlenwasserstoffen durchgeführt. Besonders bevorzugt ist Dichlormethan und 1,2-Dichlorethan. Friedel-Crafts-Reaktionen werden in einem Temperaturbereich von -30°C bis 120°C, bevorzugt von 30°C bis 100°C durchgeführt. Allerdings können die Reaktionen auch ohne Lösungsmittel durchgeführt werden. Die Reaktionen können auch in der Mikrowelle durchgeführt werden.

Verbindungen der allgemeinen Formel (5-3), in der **U, V, X, Y, R¹, R²** und **R³** wie voranstehend erwähnt definiert sind, können wie in Schema 5 gezeigt, dargestellt werden.

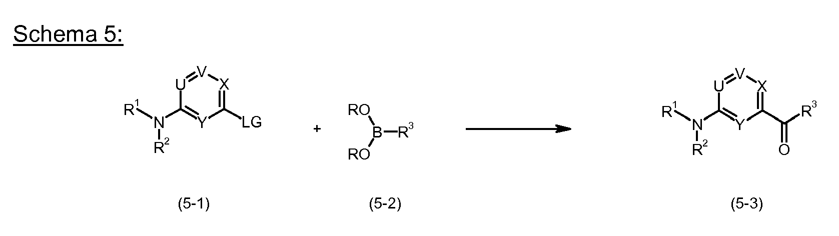

Analog zu einem Verfahren von T. Ishiyama et al. (J. Org. Chem., 1998, 63, 4726) kann eine Verbindung der allgemeinen Formel (5-1), die eine Austrittsgruppe LG besitzt, mit einer Bor-substituierten Verbindung, wie Boronsäure (R=H), Boronsäureester (R= Alkyl) Dialkylarylboran in Anwesenheit eines Katalysator und einer Base in einem inerten Lösungsmittel und einer Kohlenmonoxid-Atmosphäre, bevorzugt in einem Temperaturbereich von Raumtemperatur bis zum Rückfluss des Lösungsmittels, umgesetzt werden. Bevorzugt sind erhöhte Reaktionstemperaturen von 80°C-110°C unter erhöhtem Kohlenmonoxid-Druck. Für den Katalysator kann zusätzlich ein geeigneter Ligand verwendet werden. Es können Alkalimetalliodide wie Natriumjodid oder Kaliumjodid als Additive zugesetzt werden. Als Austrittsgruppe LG können Bromide, Iodide, Trifluoracetate, Trifluormethansulfonate, Methansulfonate und Toluolsulfonate fungieren, müssen aber nicht auf diese beschränkt sein. Als inerte Lösungsmittel können Xylol, Tetrahydrofuran, Dimethylformamid, Dimethoxyethan, Toluol, Benzol, Anisol, 1,4-Dioxan, Acetonitril oder Lösungsmittelgemische verwendet werden. Bevorzugtes Lösungsmittel ist Anisol. Geeignete Basen sind anorganische Basen wie Cäsiumcarbonat, Cäsiumacetat, Kaliumcarbonat, Natriumcarbonat oder Kaliumphosphat. Die Reaktionen werden in einer Kohlenmonoxid-Atmosphäre durchgeführt, wobei der Kohlenmonoxid-Druck 1 bis 50 bar betragen kann. Typische Katalysatoren sind z.B. Palladium-Katalysatoren wie Tris-(dibenzylidenaceton)-dipalladium(0), Tetrakis-(triphenylphosphin)-palladium(0), Palladium-(II)-acetat, Pd(PPh₃)₂Cl₂, Pd(CH₃CN)₂Cl₂, Pd(dppf)Cl₂ oder Palladium(II)-chlorid. Typische Liganden sind z.B. Triphenylphosphin, Tricyclohexylphosphin, Tri-*tert*-Butylphosphin, Triphenylarsen, BINAP, XPhos, XantPhos, oder 2-(Di-*tert*-butylphosphino)biphenyl, 1,1'-bis(Diphenylphosphino)ferrocen (Dppf), 1,2-bis(Diphenylphosphino)ethan (dppe), 1,3-bis(Diphenylphosphino)propan (dppp) und 1,4-bis(Diphenylphosphino)butan (dppb). Besonders bevorzugt ist die Verwendung von Pd(PPh₃)₂Cl₂ als Katalysator, Kaliumcarbonat als Base, 1 bar Kohlenmonoxid, Kaliumjodid als Additiv und Anisol als Lösungsmittel. Die entsprechenden Bor-substituierten Verbindungen sind entweder kommerziell erhältlich oder lassen sich aus metallierten Verbindungen durch Reaktion mit einem Bor-Elektrophil wie z.B. Borsäureester oder ein Derivat davon herstellen. Darüber hinaus können die Bor-substituierten Verbindungen aus den entsprechenden halogenierten bzw. pseudohalogenierten Vorläufer-Molekülen in einer Übergangsmetall-katalysierten Reaktion, z.B. mit Palladium und einem Diborolan bzw. Bor-Verbindung hergestellt werden (siehe Tetrahedron Lett. 2003, 4895-4898 und darin zitierte Referenzen).

Eine Synthese für Verbindungen der allgemeinen Formel (6-3), in der **U, V, X, Y** und **R³** wie voranstehend erwähnt definiert sind, ist in Schema 6 dargestellt 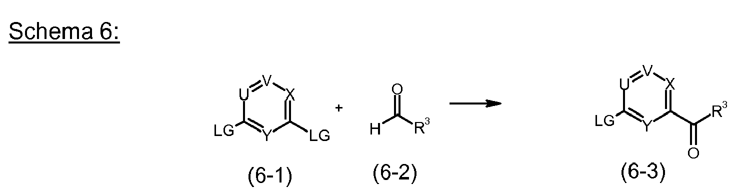 In Analogie zu einem Verfahren von A. Miyashita et al. (Heterocycles, 1997, Vol. 45, No. 11, 2159-2173) lassen sich Verbindungen der allgemeinen Formel (6-1), die eine Austrittsgruppe LG besitzen, mit aromatischen Aldehyden in Gegenwart eines Katalysators und einer Base in inerten Lösungsmitteln zu Verbindungen der allgemeinen Form (6-3) umsetzen. Als Austrittsgruppe LG können Fluoride, Chloride, Bromide, Iodide, Trifluormethansulfonate, Methansulfonate und Toluolsulfonate fungieren, müssen aber nicht auf diese beschränkt sein. Besonders bevorzugt sind Chloride und Bromide. Als inerte Lösungsmittel können cyclische Ether (bevorzugt ist Tetrahydrofuran) und Dialkylformamide (bevorzugt ist Dimethylformamid), verwendet werden. Geeignete Katalysatoren sind Azolium Salze, wie 1,3-Dimethylimidazoliumiodid oder 1,3-Dimethylbenzimidazoliumiodid. Als Base eigen sich Metallhydride. Ganz besonders bevorzugt ist Natriumhydrid. Die Reaktionen werden in einem Temperaturbereich von RT bis Rückfluss des Lösungsmittels durchgeführt. Bevorzugt sind erhöhte Temperaturen.

Die Reaktion kann auch anstelle von Azolium Salze und Base auch mit Natrium p-Tolylsulfinat in Anwesenheit eines Alkalimetallcyanids (bevorzugt Kaliumcyanid) in einem inerten Lösungsmittel bei erhöhten Temperaturen durchgeführt werden. (A. Miyashita et al., Heterocycles, 1998, Vol. 47, No. 1, 407-414).

In Analogie zu A. Miyashita et al. (Heterocycles, 1997, Vol. 45, No. 11, 2159-2173) und den darin angegebenen Literaturstellen lassen sich Verbindungen der allgemeinen Formel (7-4), in der **U, V, X, Y** und **R³** wie voranstehend erwähnt definiert sind, wie in Schema 7 dargestellt, herstellen. 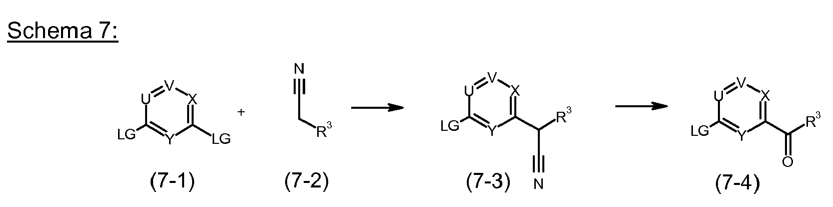 Verbindungen der allgemeinen Formel (7-1), die eine Austrittsgruppe LG besitzen, können mit 2-Arylacetonitril bzw. 2-Heteroarylacetonitril in Gegenwart einer Base in inerten Lösungsmittel zu Verbindungen der allgemeinen Form (7-3) umgesetzt werden. Als Austrittsgruppe LG können Fluoride, Chloride, Bromide, Iodide, Trifluormethansulfonate, Methansulfonate und Toluolsulfonate fungieren, müssen aber nicht auf diese beschränkt sein. Besonders bevorzugt sind Chloride und Bromide. Als inerte Lösungsmittel können Dialkylformamide (bevorzugt ist Dimethylformamid) verwendet werden. Als Base eignen sich Metallhydride. Ganz besonders bevorzugt ist Natriumhydrid. Die Reaktionen werden in einem Temperaturbereich von RT bis Rückfluss des Lösungsmittels durchgeführt. Bevorzugt sind Reaktionen bei erhöhten Temperaturen. Die Synthese der Verbindungen der allgemeinen Formel (7-4) erfolgt durch oxidative Decyanierung von Verbindungen der allgemeinen Formel (7-3). Oxidative Decyanierungen erfolgen in inerten Lösungsmitteln, durch die in Gegenwart von einer Base Sauerstoff-Gas hindurchgeleitet wird. Als inerte Lösungsmittel können cyclische Ether (bevorzugt ist Tetrahydrofuran) verwendet werden. Als Base eignen sich Metallhydride. Ganz besonders bevorzugt ist Natriumhydrid. Die Reaktionen werden in einem Temperaturbereich von -20°C bis Rückfluss des Lösungsmittels durchgeführt. Bevorzugt sind Reaktionen bei RT.

Die erfindungsgemäßen neuen Verbindungen der allgemeinen Formel **I** können ein oder mehrere Chiralitätszentren enthalten. Sind beispielsweise zwei Chiralitätszentren vorhanden, dann können die Verbindungen in Form zweier diastereomerer Antipodenpaare auftreten. Die Erfindung umfasst die einzelnen Isomere ebenso wie ihre Gemische.
Die Trennung der jeweiligen Diastereomeren gelingt auf Grund ihrer unterschiedlichen physikochemischen Eigenschaften, z.B. durch fraktionierte Kristallisation aus geeigneten Lösemitteln, durch Hochdruckflüssigkeits- oder Säulenchromatographie unter Verwendung chiraler oder bevorzugt achiraler stationärer Phasen. Die Trennung von unter die allgemeine Formel **I** fallenden Racematen gelingt beispielsweise durch HPLC an geeigneten chiralen stationären Phasen (z. B. Chiral AGP, Chiralpak AD). Racemate, die eine basische oder saure Funktion enthalten, lassen sich auch über die diastereomeren, optisch aktiven Salze trennen, die bei Umsetzung mit einer optisch aktiven Säure, beispielsweise (+)- oder (-)-Weinsäure, (+)- oder (-)-Diacetylwein-säure, (+)- oder (-)-Monomethyltartrat oder (+)- oder (-)-Camphersulfonsäure, bzw. einer optisch aktiven Base, beispielsweise mit (R)-(+)-I-Phenylethylamin, (S)-(-)-I-Phenylethyl-amin oder (S)-Brucin, entstehen.

Nach einem üblichen Verfahren zur Isomerentrennung wird das Racemat einer Verbindung der allgemeinen Formel **I** mit einer der vorstehend angegebenen optisch aktiven Säuren bzw. Basen in äquimolarer Menge in einem Lösemittel umgesetzt und die erhaltenen kristallinen, diastereomeren, optisch aktiven Salze unter Ausnutzung ihrer verschiedenen Löslichkeit getrennt. Diese Umsetzung kann in jeder Art von Lösemitteln durchgeführt werden, solange sie einen ausreichenden Unterschied hinsichtlich der Löslichkeit der Salze aufweisen. Vorzugsweise werden Methanol, Ethanol oder deren Gemische, beispielsweise im Volumenverhältnis 50:50, verwendet. Sodann wird jedes der optisch aktiven Salze in Wasser gelöst, mit einer Base, wie Natriumcarbonat oder Kaliumcarbonat, oder mit einer geeigneten Säure, beispielsweise mit verdünnter Salzsäure oder wässeriger Methansulfonsäure, vorsichtig neutralisiert und dadurch die entsprechende freie Verbindung in der (+)- oder (-)-Form erhalten.

Jeweils nur das (R)- oder (S)-Enantiomer bzw. ein Gemisch zweier optisch aktiver, unter die allgemeine Formel **I** fallender, diastereomerer Verbindungen wird auch dadurch erhalten, dass man die oben beschriebenen Synthesen mit jeweils einer geeigneten *(R)-*bzw. (S)-konfigurierten Reaktionskomponente durchführt Die neuen Verbindungen der allgemeinen Formel I und deren physiologisch verträgliche Salze weisen wertvolle pharmakologische Eigenschaften auf, die auf ihre selektiven CGRP-antagonistischen Eigenschaften zurückgehen. Ein weiterer Gegenstand der Erfindung sind diese Verbindungen enthaltende Arzneimittel, deren Verwendung und deren Herstellung.

Die voranstehend genannten neuen Verbindungen und deren physiologisch verträgliche Salze besitzen CGRP-antagonistische Eigenschaften und zeigen gute Affinitäten in CGRP-Rezeptorbindungsstudien. Die Verbindungen weisen in den nachstehend beschriebenen pharmakologischen Testsystemen CGRP-antagonistische Eigenschaften auf.

Zum Nachweis der Affinität der voranstehend genannten Verbindungen zu humanen CGRP-Rezeptoren und ihrer antagonistischen Eigenschaften wurden die folgenden Versuche durchgeführt:
A. Bindungsstudien mit (den humanen CGRP-Rezeptor exprimierenden) SK-N-MC-Zellen
   SK-N-MC-Zellen werden in "Dulbecco's modified Eagle Medium" kultiviert. Das Medium konfluenter Kulturen wird entfernt. Die Zellen werden zweimal mit PBS-Puffer (Gibco 041-04190 M) gewaschen, durch Zugabe von PBS-Puffer, versetzt mit 0.02% EDTA, abgelöst und durch Zentrifugation isoliert. Nach Resuspension in 20 ml "Balanced Salts Solution" [BSS (in mM): NaCl 120, KCl 5.4, NaHCO₃ 16.2, MgSO₄ 0.8, NaHPO₄ 1.0, CaCl₂ 1.8, D-Glucose 5.5, HEPES 30, pH 7.40] werden die Zellen zweimal bei 100 x g zentrifugiert und in BSS resuspendiert. Nach Bestimmung der Zellzahl werden die Zellen mit Hilfe eines Ultra-Turrax homogenisiert und für 10 Minuten bei 3000 x g zentrifugiert. Der Überstand wird verworfen und das Pellet in Tris-Puffer (10 mM Tris, 50 mM NaCl, 5 mM MgCl₂, 1 mM EDTA, pH 7.40), angereichert mit 1% Rinderserum-Albumin und 0.1% Bacitracin, rezentrifugiert und resuspendiert (1 ml / 1000000 Zellen). Das Homogenat wird bei -80°C eingefroren. Die Membranpräparationen sind bei diesen Bedingungen für mehr als 6 Wochen stabil.
   Nach Auftauen wird das Homogenat 1:10 mit Assay-Puffer (50 mM Tris, 150 mM NaCl, 5 mM MgCl₂, 1 mM EDTA, pH 7.40) verdünnt und 30 Sekunden lang mit einem Ultra-Turrax homogenisiert. 230 µl des Homogenats werden für 180 Minuten bei Raumtemperatur mit 50 pM ¹²⁵I-Iodotyrosyl-Calcitonin-Gene-Related Peptide (Amersham) und ansteigenden Konzentrationen der Testsubstanzen in einem Gesamtvolumen von 250 µl inkubiert. Die Inkubation wird durch rasche Filtration durch mit Polyethylenimin (0.1%) behandelte GF/B-Glasfaserfilter mittels eines Zellharvesters beendet. Die an Protein gebundene Radioaktivität wird mit Hilfe eines Gammacounters bestimmt. Als nichtspezifische Bindung wird die gebundene Radioaktivität nach Gegenwart von 1 µM humanem CGRP-alpha während der Inkubation definiert.
   Die Analyse der Konzentrations-Bindungskurven erfolgt mit Hilfe einer computergestützten nichtlinearen Kurvenanpassung.
   Die eingangs erwähnten Verbindungen zeigen in dem beschriebenen Test Ki-Werte ≤ 50 µM.
B. CGRP-Antagonismus in SK-N-MC-Zellen

SK-N-MC-Zellen (1 Mio. Zellen) werden zweimal mit 250 µl Inkubationspuffer (Hanks' HEPES, 1 mM 3-Isobutyl-1-methylxanthin, 1% BSA, pH 7.4) gewaschen und bei 37°C für 15 Minuten vorinkubiert. Nach Zugabe von CGRP (10 µl) als Agonist in steigenden Konzentrationen (10⁻¹¹ bis 10⁻⁶ M) bzw. zusätzlich von Substanz in 3 bis 4 verschiedenen Konzentrationen wird nochmals 15 Minuten inkubiert.

Intrazelluläres cAMP wird anschließend durch Zugabe von 20 µl 1M HCl und Zentrifugation (2000 x g, 4°C für 15 Minuten) extrahiert. Die Überstände werden in flüssigem Stickstoff eingefroren und beĩ 20°C gelagert.

Die cAMP-Gehalte der Proben werden mittels Radioimmunassay (Fa. Amersham) bestimmt und die pA₂-Werte antagonistisch wirkender Substanzen graphisch ermittelt.

Die erfindungsgemäßen Verbindungen zeigen in dem beschriebenen *in vitro*-Testmodell CGRP-antagonistische Eigenschaften in einem Dosisbereich zwischen 10⁻¹² bis 10⁻⁵ M.

Um zu zeigen, dass die Verbindungen der allgemeinen Formel **I** mit unterschiedlichen Strukturelementen gute bis sehr gute CGRP-antagonistische Aktivitäten zeigen, werden in der folgenden Tabelle die Kᵢ-Werte angegeben, die gemäß dem voranstehenden Versuchsprotokoll erhalten wurden. Dazu wird angemerkt, dass die Verbindungen im Hinblick auf ihre unterschiedlichen strukturellen Elemente ausgewählt wurden und nicht, um spezifische Verbindungen hervorzuheben:

| **Beispiel** | **Kᵢ [nM]** |
|---|---|
| (1) | 81 |
| (2) | 89 |
| (3) | 204 |

### INDIKATIONSGEBIETE

Aufgrund ihrer pharmakologischen Eigenschaften eignen sich die erfindungsgemäßen Verbindungen und deren Salze mit physiologisch verträglichen Säuren somit zur akuten und prophylaktischen Behandlung von Kopfschmerzen, insbesondere Migräne-, Cluster-Kopfschmerz sowie Spannungskopfschmerzen. Weiterhin beeinflussen die erfindungsgemäßen Verbindungen auch die folgenden Erkrankungen positiv: Nicht-insulinabhängigen Diabetes mellitus ("NIDDM"), cardiovaskuläre Erkrankungen, Morphintoleranz, Clostridiumtoxin-bedingte Durchfallerkrankungen, Erkrankungen der Haut, insbesondere thermische und strahlenbedingte Hautschäden inklusive Sonnenbrand, Lichen, Prurigo, pruriginöse Toxidermien sowie schwerer Juckreiz, entzündliche Erkrankungen, z.B. entzündliche Gelenkerkrankungen (Osteoarthritis, rheumatoide Arthritis, neurogene Arthritis), generalisierter Weichteilrheumatismus (Fibromyalgie), neurogene Entzündungen der oralen Mucosa, entzündliche Lungenerkrankungen, allergische Rhinitis, Asthma, COPD, Erkrankungen, die mit einer überschießenden Gefäßerweiterung und dadurch bedingter verringerter Gewebedurchblutung einhergehen, z.B. Schock und Sepsis, chronische Schmerzerkrankungen, wie z.B. diabetische Neuropathien, durch Chemotherapien induzierte Neuropathien, HIV-induzierte Neuropathien, postherpetische Neuropathien durch Gewebetrauma induzierte Neuropathien, trigeminale Neuralgien, temporomandibuläre Dysfunktionen, CRPS (complex regional pain syndrome), Rückenschmerzen, und viszerale Erkrankungen, wie z.B. irritable bowel syndrome (IBS), inflammatory bowel syndrome. Darüber hinaus zeigen die erfindungsgemäßen Verbindungen eine lindernde Wirkung auf Schmerzzustände im allgemeinen. Die Symptomatik menopausaler, durch Gefäßerweiterung und erhöhten Blutfluss verursachter Hitzewallungen östrogendefizienter Frauen sowie hormonbehandelter Prostatakarzinompatienten und Kastraten wird durch die CGRP-Antagonisten der vorliegenden Anwendung präventiv und akut-therapeutisch günstig beeinflusst, wobei sich dieser Therapieansatz vor der Hormonsubstitution durch Nebenwirkungsarmut auszeichnet.

Vorzugsweise eignen sich die erfindungsgemäßen Verbindungen zur akuten und prophylaktischen Behandlung von Migräne- und Cluster-Kopfschmerz, zur Behandlung des irritable bowel syndroms (IBS) und zur präventiven und akut-therapeutischen Behandlung von Hitzewallungen östrogendefizienter Frauen.

Die zur Erzielung einer entsprechenden Wirkung erforderliche Dosierung beträgt zweckmäßigerweise bei intravenöser oder subkutaner Gabe 0.0001 bis 3 mg/kg Körpergewicht, vorzugsweise 0.01 bis 1 mg/kg Körpergewicht, und bei oraler, nasaler oder inhalativer Gabe 0.01 bis 10 mg/kg Körpergewicht, vorzugsweise 0.1 bis 10 mg/kg Körpergewicht, jeweils ein- bis dreimal täglich.

Sofern die Behandlung mit CGRP-Antagonisten oder/und CGRP-Release-Hemmern in Ergänzung zu einer üblichen Hormonsubstitution erfolgt, empfiehlt sich eine Verringerung der vorstehend angegebenen Dosierungen, wobei die Dosierung dann 1/5 der vorstehend angegebenen Untergrenzen bis zu 1/1 der vorstehend angegebenen Obergrenzen betragen kann.

Ein weiterer Gegenstand der Erfindung ist die Verwendung der erfindungsgemäßen Verbindungen als wertvolle Hilfsmittel zur Erzeugung und Reinigung (Affinitätschromatographie) von Antikörpern sowie, nach geeigneter radioaktiver Markierung, beispielsweise durch Tritiierung geeigneter Vorstufen, beispielsweise durch katalytische Hydrierung mit Trithium oder Ersatz von Halogenatomen durch Tritium, in RIA- und ELISA-Assays und als diagnostische bzw. analytische Hilfsmittel in der Neurotransmitter-Forschung.

### KOMBINATIONEN

Als Kombinationspartner denkbare Wirkstoffklassen sind z.B. Antiemetica, Prokinetica, Neuroleptica, Antidepressiva, Neurokinin-Antagonisten, Anticonvulsiva, Histamin-H1-Rezeptorantagonisten, β-Blocker, α-Agonisten und α-Antagonisten, Ergotalkaloiden, schwachen Analgetica, nichtsteroidalen Antiphlogistica, Corticosteroiden, Calcium-Antagonisten, 5-HT_{1B/1D}-Agonisten oder andere Antimigränemitteln, die zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/- Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen, Lösungen, Dosieraerosole oder Zäpfchen eingearbeitet werden können.

Für die oben erwähnten Kombinationen kommen somit als weitere Wirksubstanzen beispielsweise die nicht-steroidalen Antiphlogistika Aceclofenac, Acemetacin, Acetylsalicylsäure, Acetaminophen (Paracetamol), Azathioprin, Diclofenac, Diflunisal, Fenbufen, Fenoprofen, Flurbiprofen, Ibuprofen, Indometacin, Ketoprofen, Leflunomid, Lornoxicam, Mefenaminsäure, Naproxen, Phenylbutazon, Piroxicam, Sulfasalazin, Zomepirac oder deren pharmazeutisch verträgliche Salze sowie Meloxicam und andere selektive COX2-Inhibitoren, wie beispielsweise Rofecoxib, Valdecoxib, Parecoxib, Etoricoxib und Celecoxib, in Betracht sowie Substanzen, die frühere oder spätere Schritte in der Prostaglandin-Synthese inhibieren oder Prostaglandinrezeptor Antagonisten wie z.B.
EP2-Rezeptor Antagonisten und IP-Rezeptor Antagonisten.

Weiterhin können Ergotamin, Dihydroergotamin, Metoclopramid, Domperidon, Diphenhydramin, Cyclizin, Promethazin, Chlorpromazin, Vigabatrin, Timolol, Isomethepten, Pizotifen, Botox, Gabapentin, Pregabalin, Duloxetin, Topiramat, Riboflavin, Montelukast, Lisinopril, Micardis, Prochlorperazin, Dexamethason, Flunarizin, Dextropropoxyphen, Meperidin, Metoprolol, Propranolol, Nadolol, Atenolol, Clonidin, Indoramin, Carbamazepin, Phenytoin, Valproat, Amitryptilin, Imipramine, Venlafaxine, Lidocain oder Diltiazem und andere 5-HT_{1B/1D}-Agonisten wie z.B. Almotriptan, Avitriptan, Eletriptan, Frovatriptan, Naratriptan, Rizatriptan, Sumatriptan und Zolmitriptan verwendet werden.

Außerdem können CGRP-Antagonisten mit Vanilloid-Rezeptor Antagonisten, wie z.B. VR-1 Antagonisten, Glutamatrezeptor Antagonisten, wie z.B. mGlu5-Rezeptor Antagonisten, mGlu1-Rezeptor Antagonisten, iGlu5-Rezeptor Antagonisten, AMPA-Rezeptor Antagonisten, Purinrezeptor Blockern, wie z.B. P2X3 Antagonisten, NO-Synthase Inhibitoren, wie z.B. iNOS Inhibitoren, Calciumkanal-Blockern, wie z.B. PQ-typ Blockern, N-typ Blockern, Kaliumkanalöffnern, wie z.B. KCNQ Kanalöffnern, Natriumkanal Blockern, wie z.B. PN3 Kanal Blockern, NMDA-Rezeptor Antagonisten, Acid-sensing lonenkanal Antagonisten, wie z.B. ASIC3 Antagonisten, Bradykinin Rezeptor Antagonisten wie z.B. B1-Rezeptor Antagonisten, Cannabinoid-Rezeptor Agonisten, wie z.B. CB2 Agonisten, CB1 Agonisten, Somatostatin-Rezeptor Agonisten, wie z.B. sst2 Rezeptor Agonisten gegeben werden.

Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung, also beispielsweise 20 bis 100 mg Sumatriptan.

### DARREICHUNGSFORMEN

Die erfindungsgemäß hergestellten Verbindungen können entweder alleine oder gegebenenfalls in Kombination mit anderen Wirksubstanzen zur Behandlung von Migräne intravenös, subkutan, intramuskulär, intraartikulär, intrarektal, intranasal, durch Inhalation, topisch, transdermal oder oral erfolgen, wobei zur Inhalation insbesondere Aerosolformulierungen geeignet sind. Die Kombinationen können entweder simultan oder sequentiell verabreicht werden.

Geeignete Anwendungsformen sind beispielsweise Tabletten, Kapseln, Lösungen, Säfte, Emulsionen oder Inhalationspulver oder -aerosole. Hierbei soll der Anteil der pharmazeutisch wirksamen Verbindung(en) jeweils im Bereich von 0.1 bis 90 Gew.-%, bevorzugt 0.5 bis 50 Gew.-% der Gesamtzusammensetzung liegen, d.h. in Mengen die ausreichend sind, um den voranstehend angegebenen Dosierungsbereich zu erreichen.

Die orale Gabe kann in Form einer Tablette, als Pulver, als Pulver in einer Kapsel (z.B. Hartgelatinekapsel), als Lösung oder Suspension erfolgen. Im Fall einer inhalativen Gabe kann die Wirkstoffkombination als Pulver, als wässrige oder wässrig-ethanolische Lösung oder mittels einer Treibgasformulierung erfolgen.

Bevorzugt sind deshalb pharmazeutische Formulierungen gekennzeichnet durch den Gehalt an einer oder mehrerer Verbindungen der Formel **I** gemäß der obigen bevorzugten Ausführungsformen.

Besonders bevorzugt ist es, wenn die Verbindungen der Formel **I** oral verabreicht werden, ganz besonders bevorzugt ist es, wenn die Verabreichung ein oder zweimal täglich erfolgt. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein Geschmack verbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt. Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuss-oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mit verwendet werden. Im Falle wässriger Suspensionen können die Wirkstoffe außer den oben genannten Hilfsstoffen mit verschiedenen Geschmacksaufbesserern oder Farbstoffen versetzt werden.

Ebenfalls bevorzugt ist es, wenn die Verbindungen der Formel **I** inhalativ verabreicht werden, besonders bevorzugt ist es, wenn die Verabreichung ein oder zweimal täglich erfolgt. Hierzu müssen die Verbindungen der Formel **I** in inhalierbaren Darreichungsformen bereitgestellt werden. Als inhalierbare Darreichungsformen kommen Inhalationspulver, treibgashaltige Dosieraerosole oder treibgasfreie Inhalationslösungen in Betracht, die gegebenenfalls im Gemisch mit gebräuchlichen physiologisch verträglichen Hilfsstoffen vorliegen.

Im Rahmen der vorliegenden Erfindung sind von dem Begriff treibgasfreie Inhalationslösungen auch Konzentrate oder sterile, gebrauchsfertige Inhalationslösungen umfasst.

Die im Rahmen der vorliegenden Erfindung einsetzbaren Darreichungsformen werden im nachfolgenden Teil der Beschreibung detailliert beschrieben.

### EXPERIMENTELLER TEIL

Für die hergestellten Verbindungen liegen in der Regel ¹H-NMR und/oder Massenspektren vor. Wenn nicht anders angegeben, werden R_{f}-Werte unter Verwendung von DC-Fertigplatten Kieselgel 60 F254 (E. Merck, Darmstadt, Artikel-Nr. 1.05714) ohne Kammersättigung bestimmt. Die bei den Fliessmitteln angegebenen Verhältnisse beziehen sich auf Volumeneinheiten der jeweiligen Lösungsmittel. Die angegebenen Volumeneinheiten bei NH₃ beziehen sich auf eine konzentrierte Lösung von NH₃ in Wasser.
Soweit nicht anders vermerkt sind die bei den Aufarbeitungen der Reaktionslösungen verwendeten Säure-, Basen- und Salzlösungen wässrige Systeme der angegebenen Konzentrationen. Zu chromatographischen Reinigungen wird Kieselgel der Firma Millipore (MATREX*^{™}*, 35 bis 70 µm) verwendet. Die angegebenen HPLC-Daten werden unter nachstehend angeführten Parametern und unter Verwendung der aufgeführten Säulen erhoben:

### Verwendete Säulen:

### (Säulentemperatur: 30°C; Injektionsvolumen: 5 µL; Detektion bei 254 nm)

| | |
|---|---|
| S1 | Zorbax-Säule (Agilent Technologies), SB (Stable Bond) C18; 3.5 µm; 4.6 x 75 mm |
| S2 | Zorbax-Säule (Agilent Technologies), SB (Stable Bond) C18; 1.8 µm; 3.0 x 30 mm |
| S3 | Zorbax-Säule (Agilent Technologies), SB (Stable Bond) C18; 5 µm; 4.6 x 75 mm |
| S4 | Zorbax-Säule (Agilent Technologies), SB (Stable Bond) C18, 7 µm; 50 x 140 mm |

### Verwendete Lösungsmittel:

Lösungsmittel A: Wasser (mit 0.1% Ameisensäure), Lösungsmittel B: Acetonitril (mit 0.1% Ameisensäure), die prozentualen Angaben beziehen sich auf das Gesamtvolumen

**Gradienten:**

| Gradient | Zeit [min] | %A | %B |
|---|---|---|---|
| **G1** (1.6 mL/min) | 0.00 | 95 | 5 |
| | 0.10 | 95 | 5 |
| | 1.75 | 5 | 95 |
| | 1.90 | 5 | 95 |
| | 1.95 | 95 | 5 |
| | 2.00 | 95 | 5 |
| **G2** (1.6 mL/min) | 0.00 | 95 | 5 |
| | 4.50 | 10 | 90 |
| | 5.00 | 10 | 90 |
| | 5.50 | 95 | 5 |
| **G3** (1.6 mL/min) | 0.00 | 95 | 5 |
| | 4.00 | 50 | 50 |
| | 4.50 | 10 | 90 |
| | 5.00 | 10 | 90 |
| | 5.50 | 95 | 5 |

**Methoden:**

| | Säule | Gradient |
|---|---|---|
| Methode A | S1 | G1 |
| Methode B | S2 | G1 |
| Methode C | S1 | G2 |
| Methode D | S1 | G3 |
| Methode E | S4 | G1 |

Bei präparativen HPLC-Reinigungen werden in der Regel die gleichen Gradienten verwendet, die bei der Erhebung der analytischen HPLC-Daten benutzt wurden. Die Sammlung der Produkte erfolgt massengesteuert, die Produkt enthaltenden Fraktionen werden vereinigt und gefriergetrocknet.

Falls nähere Angaben zur Konfiguration fehlen, bleibt offen, ob es sich um reine Enantiomere handelt oder ob partielle oder gar völlige Racemisierung eingetreten ist.

### In den Versuchsbeschreibungen werden die folgenden Abkürzungen verwendet:

- Cyc: Cyclohexan
- DCM: Dichlormethan
- DIPE: Diisopropylether
- DIPEA: Diisopropylethylamin
- DMF: *N,N*-Dimethylformamid
- d. Th.: der Theorie
- EtOAc: Essigsäureethylester
- EtOH: Ethanol
- FM: Fließmittel
- HCl: Salzsäure
- HCOOH: Ameisensäure
- AcOH: Essigsäure
- i.vac.: in vacuo (im Vakuum)
- MeOH: Methanol
- NaOH: Natriumhydroxid
- NH₄OH: Ammoniumhydroxid (wässrige Ammoniak-Lösung, 30%)
- PE: Petrolether
- RT: Raumtemperatur
- THF: Tetrahydrofuran
- ULTS: Umluft-Trockenschrank

### Herstellung der Ausgangsverbindungen

### Intermediat 1

### 6-Chlorpyrimidin-4-carbonsäurechlorid

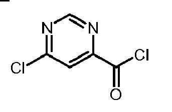

### Stufe 1: 6-Hydroxypyrimidin-4-carbonsäure 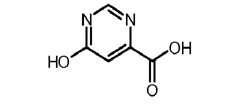

Zu 24.1 g (0.597 mol) NaOH in 3.6 L Wasser wurden 63.5 g (287 mmol) Natriumdiethyl-Oxalacetat und 30.2 g (287 mmol) Formamidin-Acetat zugegeben. Der Ansatz wurde über Nacht bei RT gerührt. Anschließend fügte man Aktivkohle hinzu und kochte den Ansatz für 1 h unter Rückfluss. Es wurde heiß abfiltriert und nach dem Erkalten mit wässriger HCl angesäuert. Die Lösung wurde bis zur Trockne einrotiert. Der Rückstand enthielt das gewünschte Produkt und wurde ohne weitere Aufreinigung in die nächste Stufe eingesetzt.

| | |
|---|---|
| Ausbeute: | 83.0 g |

### Stufe 2: 6-Chlorpyrimidin-4-carbonsäurechlorid

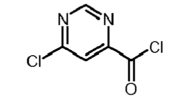

50 g (0.352 mol) 6-Hydroxypyrimidin-4-carbonsäure wurde vorgelegt und 500 mL Phosphoroxychlorid zugegeben. Anschließend wurde unter Rühren 150 g (0.720 mol) Phosphorpentachlorid portionsweise zugegeben. Der Reaktionsansatz wurde 5 h unter Rückfluss gekocht. Das Phosphoroxychlorid wurde abdestilliert und der Rückstand durch Vakuumdestillation über eine Kolonne aufgereinigt.

| | |
|---|---|
| Ausbeute: | 51.9 (83% d. Th.) |
| ESI-MS: | m/z = 176 / 178 / 180 (M)⁺ (2 Cl) |

### Intermediat 2

### (6-Chlorpyrimidin-4-yl)-(1-methyl-1H-indol-3-yl)-methanon

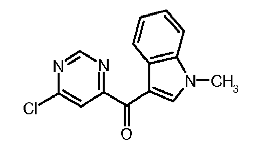

0.870 g (6.52 mmol) Aluminiumtrichlorid wurden in 12 mL DCM vorgelegt. Dazu wurde eine Lösung aus 1.00 g (5.60 mmol) 6-Chlorpyrimidin-4-carbonylchlorid in 6 mL DCM gegeben und das Gemisch 30 min bei Raumtemperatur gerührt. Anschließend wurde eine Lösung aus 0.745 mL (5.65 mmol) 1-Methylindol in 6 mL DCM langsam zugetropft. Die Reaktion wurde 1 h bei 40°C gerührt, dann auf Raumtemperatur abgekühlt, mit 15%iger (w/v) Kaliumcarbonatlösung versetzt. Das Produkt wurde mit DCM extrahiert, die organischen Phasen vereinigt und über Magnesiumsulfat getrocknet. Nach Entfernen des Lösungsmittels i.vac. wurde der erhaltene Rückstand in einem Gemisch aus PE/EtOAc (1:1) verrührt und anschließend abgesaugt und getrocknet.

| | |
|---|---|
| Ausbeute: | 0.950 g (62% d. Th.) |
| ESI-MS: | m/z = 272 / 274 (M+H)⁺ (Cl) |
| R_{f}: | 0.42 (Kieselgel, PE/EtOAc = 2:1) |

### Intermediat 3 Spiro[benzo[d][1,3]oxazin-4,4'-piperidin]-2(1H)-on-hydrochlorid

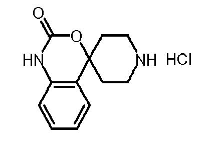

11.4 g (35.8 mmol) *tert*-Butyl-2-oxo-1,2-dihydrospiro[benzo[d][1,3]oxazin-4,4'-piperidin]-1 '-carboxylat wurden in 250 mL wässriger Salzsäure (1.3 M) über Nacht bei RT gerührt. Das Reaktionsgemisch wurde i.vac. eingeengt und das als Feststoff angefallene Rohprodukt mit Isopropanol und DIPE verrieben, abgesaugt und getrocknet.

| | |
|---|---|
| Ausbeute: | 8.50 g (93% d. Th.) |
| ESI-MS: | m/z = 219 (M+H)⁺ |
| R_{f} = | 0.14 (Kieselgel, DCM/MeOH /Cyc/NH₄OH = 70:15:15:2) |

### Herstellung der Endverbindungen

### Beispiel 1:

### 1-(6-(1-Methyl-1H-indol-3-carbonyl)pyrimidin-4-yl)-1'H-spiro[piperidin-4,4'-chinazolin]-2'(3'H)-on

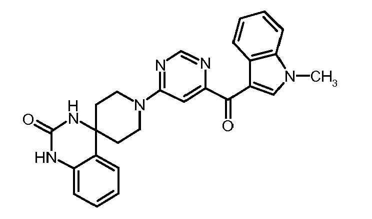

80 mg (0.29 mmol) (6-Chlorpyrimidin-4-yl)-(1-methyl-1*H*-indol-3-yl)-methanon, 64 mg (0.29 mmol) 1'*H*-Spiro[piperidin-4,4'-chinazolin]-2'(3'*H*)-on und 0.056 mL (0.32 mmol) DIPEA wurden in 2.0 mL DMF zusammen gegeben und über Nacht bei RT gerührt. Das Lösungsmittel wurde i.vac. entfernt, der verbliebene Rückstand mit Wasser versetzt und der entstandene Niederschlag abgesaugt. Das Rohprodukt wurde mittels präparativer HPLC gereinigt, die Produkt enthaltenden Fraktionen vereinigt und i.vac. eingeengt. Durch Zugabe von NaOH-Lösung fiel das Produkt als Natriumsalz aus. Dieses wurde abfiltriert und getrocknet.

| | |
|---|---|
| Ausbeute: | 105 mg (als Natriumsalz) (79% d. Th.) |
| ESI-MS: | m/z = 453 (M+H)⁺ |
| Rₜ(HPLC) = | 1.19 min (Methode E) |

### Beispiel 2:

### 5-(6-(1-Methyl-1H-indol-3-carbonyl)pyrimidin-4-ylamino)-1,3-dihydrospiro[inden-2,3'-pyrrolo[2,3-b]pyridin]-2'(1'H)-on

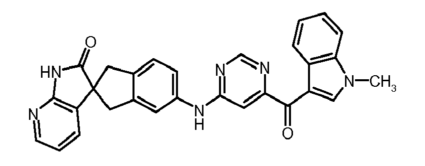

80 mg (0.29 mmol) (6-Chlorpyrimidin-4-yl)-(1-methyl-1*H*-indol-3-yl)-methanon, 88 mg (0.35 mmol) 5-Amino-1,3-dihydrospiro[inden-2,3'-pyrrolo[2,3-b]pyridin]-2'(1'*H*)-on und 9.4 mg (0.060 mmol) Benzolsulfonsäure wurden in 5.0 mL 2-Pentanol zusammen gegeben und über Nacht unter Rückfluss gerührt. Das Reaktionsgemisch wurde i.vac. eingeengt, der Rückstand in DMF aufgenommen und mittels präparativer HPLC gereinigt. Die Produkt enthaltenden Fraktionen wurden vereinigt und eingeengt.

| | |
|---|---|
| Ausbeute: | 55 mg (38% d. Th.) |
| ESI-MS: | m/z = 487 (M+H)⁺ |
| Rₜ(HPLC) = | 1.37 min (Methode E) |

### Beispiel 3:

### 1'-(6-(1-Methyl-1H-indol-3-carbonyl)pyrimidin-4-yl)spiro[benzo[d][1,3]oxazin-4,4'-piperidin]-2(1H)-on

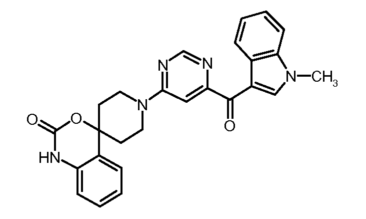

80 mg (0.29 mmol) (6-Chlorpyrimidin-4-yl)-(1-methyl-1*H*-indol-3-yl)-methanon, 75 mg (0.29 mmol) Spiro[benzo[d][1,3]oxazin-4,4'-piperidin]-2(1*H*)-on-hydrochlorid und 0.056 mL (0.32 mmol) DIPEA wurden in 2.0 mL DMF zusammen gegeben und über Nacht bei RT gerührt. Das Lösungsmittel wurde i.vac. entfernt, der verbliebene Rückstand mit Wasser versetzt und der entstandene Niederschlag abgesaugt. Das Rohprodukt wurde mittels präparativer HPLC gereinigt, die Produkt enthaltenden Fraktionen vereinigt und i.vac. eingeengt. Der Rückstand wurde in Kaliumcarbonat-Lösung aufgenommen und mit Dichlormethan extrahiert. Die vereinigten organischen Phasen wurden über Natriumsulfat getrocknet, filtriert und eingeengt.

| | |
|---|---|
| Ausbeute: | 45 mg (32% d. Th.) |
| ESI-MS: | m/z = 454 (M+H)⁺ |
| Rₜ (HPLC) = | 1.23 min (Methode E) |

Die nachfolgenden Beispiele beschreiben die Herstellung pharmazeutischer Anwendungsformen, die als Wirkstoff eine beliebige Verbindung der allgemeinen Formel **I** enthalten:

### Beispiel I

### Kapseln zur Pulverinhalation mit 1 mg Wirkstoff

**Zusammensetzung:**

| 1 Kapsel zur Pulverinhalation enthält: | |
|---|---|
| Wirkstoff | 1.0 mg |
| Milchzucker | 20.0 mg |
| Hartgelatinekapseln | 50.0 mg |
| | 71.0 mg |

### Herstellungsverfahren:

Der Wirkstoff wird auf die für Inhalativa erforderliche Korngröße gemahlen. Der gemahlene Wirkstoff wird mit dem Milchzucker homogen gemischt. Die Mischung wird in Hartgelatinekapseln abgefüllt.

### Beispiel II

### Inhalationslösung für Respimat^{®} mit 1 mg Wirkstoff

**Zusammensetzung:**

| 1 Hub enthält: | |
|---|---|
| Wirkstoff | 1.0 mg |
| Benzalkoniumchlorid | 0.002 mg |
| Dinatriumedetat | 0.0075 mg |
| Wasser gereinigt ad | 15.0 µl |

### Herstellungsverfahren:

Der Wirkstoff und Benzalkoniumchlorid werden in Wasser gelöst und in Respimat^{®}-Kartuschen abgefüllt.

### Beispiel III

Inhalationslösung für Vernebler mit 1 mg Wirkstoff

**Zusammensetzung:**

| 1 Fläschchen enthält: | |
|---|---|
| Wirkstoff | 0.1 g |
| Natriumchlorid | 0.18 g |
| Benzalkoniumchlorid | 0.002 g |
| Wasser gereinigt ad | 20.0 ml |

### Herstellungsverfahren:

### Wirkstoff, Natriumchlorid und Benzalkoniumchlorid werden in Wasser gelöst.

### Beispiel IV

### Treibgas-Dosieraerosol mit 1 mg Wirkstoff

**Zusammensetzung:**

| 1 Hub enthält: | |
|---|---|
| Wirkstoff | 1.0 mg |
| Lecithin | 0.1 % |
| Treibgas ad | 50.0 µl |

### Herstellungsverfahren:

Der mikronisierte Wirkstoff wird in dem Gemisch aus Lecithin und Treibgas homogen suspendiert. Die Suspension wird in einen Druckbehälter mit Dosierventil abgefüllt.

### Beispiel V

### Nasalspray mit 1 mg Wirkstoff

**Zusammensetzung:**

| | |
|---|---|
| Wirkstoff | 1.0 mg |
| Natriumchlorid | 0.9 mg |
| Benzalkoniumchlorid | 0.025 mg |
| Dinatriumedetat | 0.05 mg |
| Wasser gereinigt ad | 0.1 ml |

### Herstellungsverfahren:

Der Wirkstoff und die Hilfsstoffe werden in Wasser gelöst und in ein entsprechendes Behältnis abgefüllt.

### Beispiel VI

Injektionslösung mit 5 mg Wirksubstanz pro 5 ml

**Zusammensetzung:**

| | |
|---|---|
| Wirksubstanz | 5 mg |
| Glucose | 250 mg |
| Human-Serum-Albumin | 10 mg |
| Glykofurol | 250 mg |
| Wasser für Injektionszwecke ad | 5 ml |

### Herstellung:

Glykofurol und Glucose in Wasser für Injektionszwecke auflösen (Wfl); Human-Serum-Albumin zugeben; Wirkstoff unter Erwärmen auflösen; mit Wfl auf Ansatzvolumen auffüllen; unter Stickstoff-Begasung in Ampullen abfüllen.

### Beispiel VII

### Injektionslösung mit 100 mg Wirksubstanz pro 20 ml

**Zusammensetzung:**

| | |
|---|---|
| Wirksubstanz | 100 mg |
| Monokaliumdihydrogenphosphat = KH₂PO₄ | 12 mg |
| Dinatriumhydrogenphosphat = Na₂HPO₄*2H₂O | 2 mg |
| Natriumchlorid | 180 mg |
| Human-Serum-Albumin | 50 mg |
| Polysorbat 80 | 20 mg |
| Wasser für Injektionszwecke ad | 10 ml |

### Herstellung:

Polysorbat 80, Natriumchlorid, Monokaliumdihydrogenphosphat und Dinatriumhydrogenphosphat in Wasser für Injektionszwecke (Wfl) auflösen; Human-Serum-Albumin zugeben; Wirkstoff unter Erwärmen auflösen; mit Wfl auf Ansatzvolumen auffüllen; in Ampullen abfüllen.

### Beispiel VIII

### Lyophilisat mit 10 mg Wirksubstanz

**Zusammensetzung:**

| | |
|---|---|
| Wirksubstanz | 10 mg |
| Mannit | 300 mg |
| Human-Serum-Albumin | 20 mg |

| | |
|---|---|
| Wasser für Injektionszwecke ad | 2 ml |

### Herstellung:

Mannit in Wasser für Injektionszwecke (Wfl) auflösen; Human-Serum-Albumin zugeben; Wirkstoff unter Erwärmen auflösen; mit Wfl auf Ansatzvolumen auffüllen; in Vials abfüllen; gefriertrocknen.

| Lösungsmittel für Lyophilisat: | |
|---|---|
| Polysorbat 80 = Tween 80 | 20 mg |
| Mannit | 200 mg |
| Wasser für Injektionszwecke ad | 10 ml |

### Herstellung:

Polysorbat 80 und Mannit in Wasser für Injektionszwecke (Wfl) auflösen; in Ampullen abfüllen.

### Beispiel IX

### Tabletten mit 20 mg Wirksubstanz

**Zusammensetzung:**

| | |
|---|---|
| Wirksubstanz | 20 mg |
| Lactose | 120 mg |
| Maisstärke | 40 mg |
| Magnesiumstearat | 2 mg |
| Povidon K 25 | 18 mg |

### Herstellung:

Wirksubstanz, Lactose und Maisstärke homogen mischen; mit einer wässrigen Lösung von Povidon granulieren; mit Magnesiumstearat mischen; auf einer Tablettenpresse abpressen; Tablettengewicht 200 mg.

### Beispiel X

### Kapseln mit 20 mg Wirksubstanz

**Zusammensetzung:**

| | |
|---|---|
| Wirksubstanz | 20 mg |
| Maisstärke | 80 mg |
| Kieselsäure, hochdispers | 5 mg |
| Magnesiumstearat | 2.5 mg |

### Herstellung:

Wirksubstanz, Maisstärke und Kieselsäure homogen mischen; mit Magnesiumstearat mischen; Mischung auf einer Kapselfüllmaschine in Hartgelatine-Kapseln Größe 3 abfüllen.

### Beispiel XI

Zäpfchen mit 50 mg Wirksubstanz

**Zusammensetzung:**

| | |
|---|---|
| Wirksubstanz | 50 mg |
| Hartfett (Adeps solidus) q.s. ad | 1700 mg |

### Herstellung:

Hartfett bei ca. 38°C aufschmelzen; gemahlene Wirksubstanz im geschmolzenen Hartfett homogen dispergieren; nach Abkühlen auf ca. 35°C in vorgekühlte Formen ausgießen.

### Beispiel XII

Injektionslösung mit 10 mg Wirksubstanz pro 1 mL

**Zusammensetzung:**

| | |
|---|---|
| Wirksubstanz | 10 mg |
| Mannitol | 50 mg |
| Human-Serum-Albumin | 10 mg |
| Wasser für Injektionszwecke ad | 1 ml |

### Herstellung:

Mannitol in Wasser für Injektionszwecke auflösen (Wfl); Human-Serum-Albumin zugeben; Wirkstoff unter Erwärmen auflösen; mit Wfl auf Ansatzvolumen auffüllen; unter Stickstoff-Begasung in Ampullen abfüllen.

## Patentansprüche

1. Verbindungen der allgemeinen Formel **I** 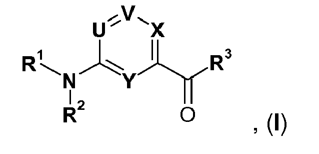 in der
**R**¹ eine Gruppe der allgemeinen Formel **IIa** oder **IIb** 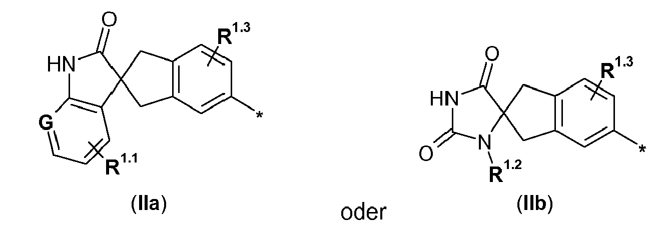 und
**R**² H oder C₁₋₃-Alkyl, oder **R**¹ und **R²** zusammen mit dem Stickstoffatom, an das sie gebunden sind, eine Gruppe der allgemeinen Formeln **IIIa** oder **IIIb** 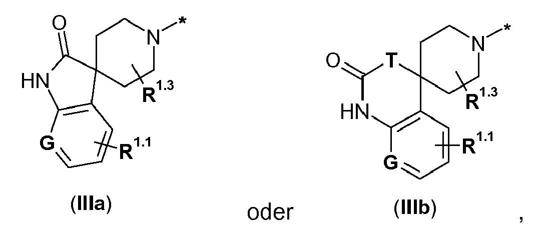
**G** C-**R**^{1.1} oder N,
**T** N-**R**^{1.2} oder O,
**R**^{1.1} unabhängig voneinander
(a) H (b) Halogen, C₁₋₃-Alkyl, -OH, -CN, -O-C₁₋₃-Alkyl, -C(O)-O-C₁₋₃-Alkyl, C₂₋₄-Alkenyl, C₂₋₄-Alkinyl, C₁₋₃-Alkyl-S-, Cyclopropyl, -NH₂, -COOH, -NH-C(O)-O-C₁₋₃-Alkyl, -NH-C(O)-C₁₋₃-Alkyl, (c) eine C₁₋₃-Alkyl- oder C₁₋₃-Alkyl-O-Gruppe, in der jede Methylengruppe mit bis zu zwei Fluoratomen und jede Methylgruppe mit bis zu drei Fluoratomen substituiert ist,
**R**^{1.2} unabhängig voneinander (a) H oder (b) C₁₋₃-Alkyl,
**R**^{1.3} (a) H, (b) F, -CN, C₁₋₃-Alkyl, -CO₂**R**^{1.3.1} oder (c) eine C₁₋₃-Alkylgruppe, in der jede Methylengruppe mit bis zu zwei Fluoratomen und jede Methylgruppe mit bis zu drei Fluoratomen substituiert sein kann,
**R**^{1.3.1} (a) H, (b) C₁₋₆-Alkyl,
**R**³ eine Gruppe ausgewählt aus (a) eine mit einem Rest **R**^{3.1} substituierte 5-gliedrige Heteroarylgruppe, die über ein Kohlenstoffatom angebunden ist, ein oder zwei Heteroatome ausgewählt aus der Gruppe N, O und S trägt und mit einer Phenylgruppe oder mit einer 5-oder 6-gliedrigen Carbocyclylgruppe kondensiert ist, wobei die Phenylgruppe zusätzlich mit den Resten **R**^{3.2}, **R**^{3.3}, **R**^{3.4} und **R**^{3.5} substituiert ist und die Carbocyclylgruppe zusätzlich mit den Resten **R**^{3.2} und **R**^{3.3} substituiert ist, oder (b) eine mit einem Rest **R**^{3.1} substituierte 5-gliedrige Heteroarylgruppe, die über ein Kohlenstoffatom angebunden ist, ein oder zwei Heteroatome ausgewählt aus der Gruppe N, O und S trägt und mit einer weiteren 5- bis 6-gliedrigen Heteroaryl- oder Heterocyclylgruppe kondensiert ist, wobei die ankondensierte Heteroarylgruppe mit einem oder zwei Resten **R**^{3.2} substituiert sein kann, **R**^{3.1} (a) H, (b) -C₁₋₆-Alkylen-**R**^{3.1.1}, -C(O)-C₁₋₃-Alkyl,
**R**^{3.1.1} (a) H, (b) -CN, -C(O)-O-**R**^{3.1.1.1}, -O-**R**^{3.1.1.1}, -N(**R**^{3.1.1.1})₂, (c) eine 5- bis 7-gliedrige Heterocyclylgruppe, die an einem oder zwei Kohlenstoffatomen mit einem oder zwei Resten **R**^{3.1.1.2} substituiert sein kann, (d) eine -C(O)-Heterocyclylgruppe, die an einem oder zwei Kohlenstoffatomen mit einem oder zwei Resten **R**^{3.1.1.2} substituiert sein kann,
**R**^{3.1.1.1} unabhängig voneinander (a) H, (b) C₁₋₆-Alkyl,
**R**^{3.1.1.2} unabhängig voneinander (a) H, (b) F, C₁₋₃-Alkyl,
**R**^{3.2} (a) H, (b) Halogen, -CN-, OH, -O-C₁₋₃-Alkyl, C₁₋₃-Alkyl, -C(O)-O-**R**^{3.2.1}, -**N**R^{3.2.1}**R**^{3.2.1}, -NH-C(O)-C₁₋₃-Alkyl, -C(O)-N**R**^{3.2.1}**R**^{3.2.1}, -S(O₂)-N**R**^{3.2.1}**R**^{3.2.1}, -O-C(O)-N**R**^{3.2.1}**R**^{3.2.1}, -N**R**^{3.2.1}-S(O)₂-C₁₋₃-Alkyl, C₂₋₃-Alkenyl, C₂₋₄-Alkinyl, -S-C₁₋₃-Alkyl, (c) eine C₁₋₃-Alkyl- oder C₁₋₃-Alkyl-O-Gruppe, in der jede Methylengruppe mit bis zu zwei Fluoratomen und jede Methylgruppe mit bis zu drei Fluoratomen substituiert ist,
**R**^{3.2.1} unabhängig voneinander (a) H, (b) C₁₋₆-Alkyl oder (c) zwei **R**^{3.2.1} Reste zusammen mit dem Atom, an das sie gebunden sind, auch einen Ring ausbilden können, der ausgewählt ist aus der Gruppe bestehend aus Azetidinyl, Pyrrolidinyl, Piperidinyl, Piperazinyl und Morpholinyl,
**R**^{3.3} (a) H, (b) Halogen, -CN, -OH, -O-C₁₋₃-Alkyl, C₁₋₃-Alkyl, -C(O)-O-**R**^{3.3.1}, -N**R**^{3.3.1}**R**^{3.3.1},
-NH-C(O)-C₁₋₃-Alkyl, -C(O)-N**R**^{3.3.1}R^{3.3.1}, -S(O₂)-N**R**^{3.3.1}**R**^{3.3.1}, -O-C(O)-N**R**^{3.3.1}**R**^{3.3.1}, -N**R**^{3.3.1}-S(O)₂-C₁₋₃-Alkyl, C₂₋₃-Alkenyl, C₂₋₄-Alkinyl, -S-C₁₋₃-Alkyl, (c) eine C₁₋₃-Alkyl- oder C₁₋₃-Alkyl-O-Gruppe, in der jede Methylengruppe mit bis zu zwei Fluoratomen und jede Methylgruppe mit bis zu drei Fluoratomen substituiert ist,
**R**^{3.3.1} unabhängig voneinander (a) H, (b) C₁₋₆-Alkyl oder (c) zwei **R**^{3.3.1} Reste zusammen mit dem Atom, an das sie gebunden sind, auch einen Ring ausbilden können, der ausgewählt ist aus der Gruppe bestehend aus Azetidinyl, Pyrrolidinyl, Piperidinyl, Piperazinyl und Morpholinyl,
**R**^{3.4} (a) H, (b) Halogen, -CN, -OH, -O-C₁₋₃-Alkyl, C₁₋₃-Alkyl, -C(O)-O-**R**^{3.4.1}, -N**R**^{3.4.1}**R**^{3.4.1}, -NH-C(O)-C₁₋₃-Alkyl, -C(O)-N**R**^{3.4.1}**R**^{3.4.1}, -S(O₂)-N**R**^{3.4.1}**R**^{3.4.1}, -O-C(O)-N**R**^{3.4.1}**R**^{3.4.1}, -N**R**^{3.4.1}-S(O)₂-C₁₋₃-Alkyl, C₂₋₃-Alkenyl, C₂₋₄-Alkinyl, -S-C₁₋₃-Alkyl, (c) eine C₁₋₃-Alkyl- oder C₁₋₃-Alkyl-O-Gruppe, in der jede Methylengruppe mit bis zu zwei Fluoratomen und jede Methylgruppe mit bis zu drei Fluoratomen substituiert ist,
**R**^{3.4.1} unabhängig voneinander (a) H, (b) C₁₋₆-Alkyl oder (c) zwei **R**^{3.4.1} Reste zusammen mit dem Atom, an das sie gebunden sind, auch einen Ring ausbilden können, der ausgewählt ist aus der Gruppe bestehend aus Azetidinyl, Pyrrolidinyl, Piperidinyl, Piperazinyl und Morpholinyl,
**R**^{3.5} (a) H, (b) Halogen, -CN, -OH, -O-C₁₋₃-Alkyl, C₁₋₃-Alkyl, -C(O)-O-**R**^{3.5.1}, -N**R**^{3.5.1}**R**^{3.5.1}, -NH-C(O)-C₁₋₃-Alkyl, -C(O)-N**R**^{3.5.1}**R**^{3.5.1}, -S(O₂)-N**R**^{3.5.1}**R**^{3.5.1}, -O-C(O)-N**R**^{3.5.1}**R**^{3.5.1}, -N**R**^{3.5.1}-S(O)₂-C₁₋₃-Alkyl, C₂₋₃-Alkenyl, C₂₋₄-Alkinyl,
-S-C₁₋₃-Alkyl, (c) eine C₁₋₃-Alkyl- oder C₁₋₃-Alkyl-O-Gruppe, in der jede Methylengruppe mit bis zu zwei Fluoratomen und jede Methylgruppe mit bis zu drei Fluoratomen substituiert ist,
**R**^{3.5.1} unabhängig voneinander (a) H, (b) C₁₋₆-Alkyl oder (c) zwei **R**^{3.5.1} Reste zusammen mit dem Atom, an das sie gebunden sind, auch einen Ring ausbilden können, der ausgewählt ist aus der Gruppe bestehend aus Azetidinyl, Pyrrolidinyl, Piperidinyl, Piperazinyl und Morpholinyl,
**U** N, N-Oxid oder CH,
**V** N oder C-**R**⁴,
**X** N, N-Oxid oder CH,
**Y** N oder C-**R**⁵,
wobei höchstens drei der voranstehend genannten Reste **U, V, X** oder **Y** gleichzeitig ein Stickstoffatom darstellen,
**R**⁴ H, -N**R**^{4.1}**R**^{4.2} oder -O-C₁₋₃-Alkyl,
**R**^{4.1} H oder C₁₋₆-Alkyl,
**R**^{4.2} H oder -SO₂C₁₋₃-Alkyl,
**R**⁵ H, Halogen oder C₁₋₃-Alkyl bedeuten, deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

2. Verbindungen der allgemeinen Formel **I** gemäß Anspruch 1, in denen **U, V, X, Y** und **R**³ wie in Anspruch 1 definiert sind und
**R**¹ eine Gruppe ausgewählt aus 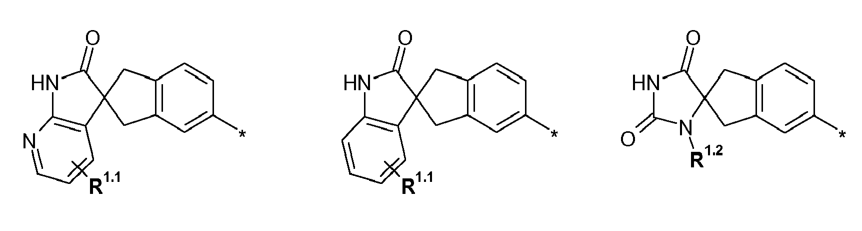 und
**R**² H oder **R**¹ und **R**² zusammen mit dem Stickstoffatom, an das sie gebunden sind, eine Gruppe ausgewählt aus 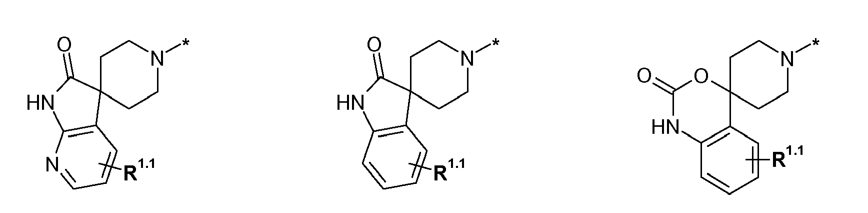 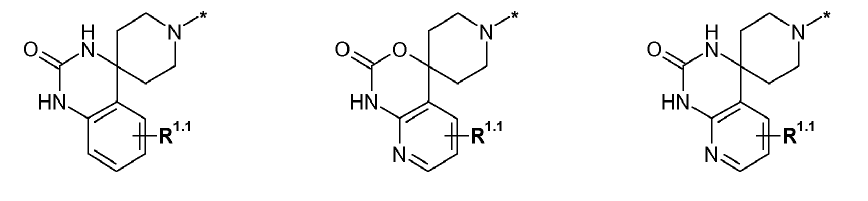
**R**^{1.1} unabhängig voneinander (a) H, (b) Halogen, C₁₋₃-Alkyl, -OH, -CN, -O-C₁₋₃-Alkyl, -C(O)-O-C₁₋₃-Alkyl, C₂₋₄-Alkenyl, C₂₋₄-Alkinyl, C₁₋₃-Alkyl-S-, -NH₂, (c) eine C₁₋₃-Alkyl- oder C₁₋₃-Alkyl-O-Gruppe, in der jede Methylengruppe mit bis zu zwei Fluoratomen und jede Methylgruppe mit bis zu drei Fluoratomen substituiert ist,
**R**^{1.2} unabhängig voneinander (a) H oder (b) CH₃ und
**R**^{1.3} (a) H, (b) F, -CN, -CO₂H, -C(O)-O-C₁₋₃-Alkyl oder (c) -CF₃ bedeutet, deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

3. Verbindungen der allgemeinen Formel **I** gemäß Anspruch 1, in denen **U, V, X, Y** und **R**³ wie in Anspruch 1 definiert sind und
**R**¹ eine Gruppe ausgewählt aus 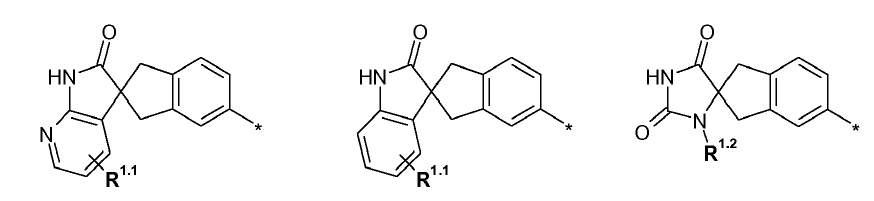 und
**R**² H oder **R**¹ und **R**² zusammen mit dem Stickstoffatom, an das sie gebunden sind, eine Gruppe ausgewählt aus 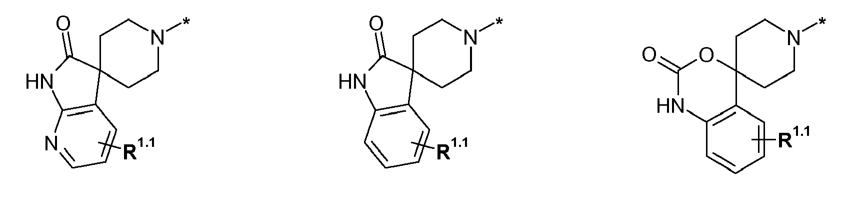 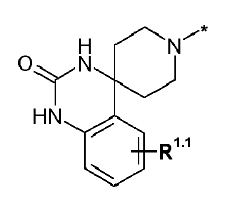
**R**^{1.1} unabhängig voneinander (a) F, CH₃, -OH, -O-CH₃ oder (b) CF₃ und
**R**^{1.2} unabhängig voneinander (a) H oder (b) CH₃ bedeutet, deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

4. Verbindungen der allgemeinen Formel I gemäß Anspruch 1, in denen **U, V, X, Y** und **R**³ wie in Anspruch 1 definiert sind und
**R**¹ eine Gruppe ausgewählt aus 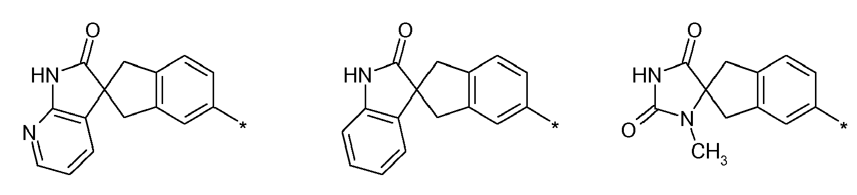 und
**R**² H oder **R**¹ und **R**² zusammen mit dem Stickstoffatom, an das sie gebunden sind, eine Gruppe ausgewählt aus 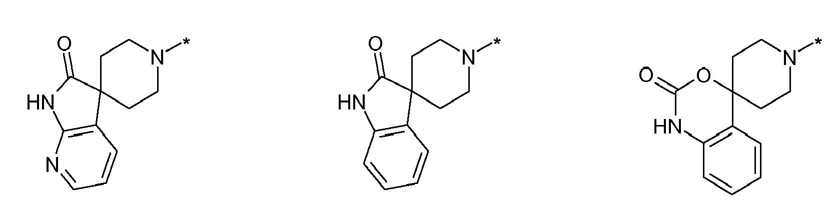 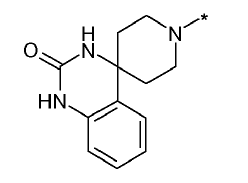 bedeutet, deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

5. Verbindungen der allgemeinen Formel **I** gemäß Anspruch 1, in denen **U, V, X, Y,**
**R**¹ und **R**² wie in Anspruch 1 definiert sind und
**R**³ eine Gruppe der allgemeinen Formeln **IV** 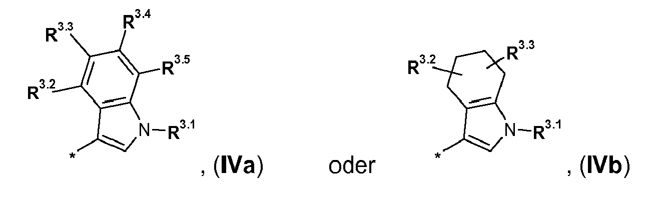
**R**^{3.1} (a) H, (b) -C₁₋₆-Alkylen-**R**^{3.1.1}, -C(O)-C₁₋₃-Alkyl,
**R**^{3.1.1} (a) H, (b) -C(O)-O-**R**^{3.1.1.1}, -O-**R**^{3.1.1.1}, -N(**R**^{3.1.1.1})₂, (c) eine 5- bis 7-gliedrige Heterocyclylgruppe, die an einem oder zwei Kohlenstoffatomen mit einem oder zwei Resten **R**^{3.1.1.2} substituiert sein kann, (d) eine -C(O)-Heterocyclylgruppe, die an einem oder zwei Kohlenstoffatomen mit einem oder zwei Resten **R**^{3.1.1.2} substituiert sein kann,
**R**^{3.1.1.1} unabhängig voneinander H, C₁₋₆-Alkyl und **R**^{3.1.1.2} unabhängig voneinander H, F, C₁₋₃-Alkyl,
**R**^{3.2} (a) H, (b) Halogen, -CN-, OH, -O-C₁₋₃-Alkyl, C₁₋₃-Alkyl, -C(O)-O-**R**^{3.2.1}, C₂₋₃-Alkenyl, C₂₋₄-Alkinyl, -S-C₁₋₃-Alkyl, (c) eine C₁₋₃-Alkyl- oder C₁₋₃-Alkyl-O-Gruppe, in der jede Methylengruppe mit bis zu zwei Fluoratomen und jede Methylgruppe mit bis zu drei Fluoratomen substituiert ist,
**R**^{3.2.1} H, C₁₋₆-Alkyl,
**R**^{3.3} (a) H, (b) Halogen, -CN, -OH, -O-C₁₋₃-Alkyl, C₁₋₃-Alkyl, -C(O)-O-**R**^{3.3.1}, C₂₋₃-Alkenyl, C₂₋₄-Alkinyl, -S-C₁₋₃-Alkyl, (c) eine C₁₋₃-Alkyl- oder C₁₋₃-Alkyl-O-Gruppe, in der jede Methylengruppe mit bis zu zwei Fluoratomen und jede Methylgruppe mit bis zu drei Fluoratomen substituiert ist,
**R**^{3.3.1} H, C₁₋₆-Alkyl,
**R**^{3.4} (a) H, (b) Halogen, -CN, -OH, -O-C₁₋₃-Alkyl, C₁₋₃-Alkyl, -C(O)-O-**R**^{3.4.1}, C₂₋₃-Alkenyl, C₂₋₄-Alkinyl, -S-C₁₋₃-Alkyl, (c) eine C₁₋₃-Alkyl- oder C₁₋₃-Alkyl-O-Gruppe, in der jede Methylengruppe mit bis zu zwei Fluoratomen und jede Methylgruppe mit bis zu drei Fluoratomen substituiert ist,
**R**^{3.4.1} H, C₁₋₆-Alkyl,
**R**^{3.5} (a) H, (b) Halogen, -CN, -OH, -O-C₁₋₃-Alkyl, C₁₋₃-Alkyl, -C(O)-O-**R**^{3.5.1}, C₂₋₃-Alkenyl, C₂₋₄-Alkinyl, -S-C₁₋₃-Alkyl, (c) eine C₁₋₃-Alkyl- oder C₁₋₃-Alkyl-O-Gruppe, in der jede Methylengruppe mit bis zu zwei Fluoratomen und jede Methylgruppe mit bis zu drei Fluoratomen substituiert ist,
**R**^{3.5.1} H, C₁₋₆-Alkyl bedeutet, deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

6. Verbindungen der allgemeinen Formel **I** gemäß Anspruch 1, in denen **U, V, X, Y, R**¹ und **R**² wie in Anspruch 1 definiert sind und
**R**³ eine Gruppe der allgemeinen Formeln **IV** 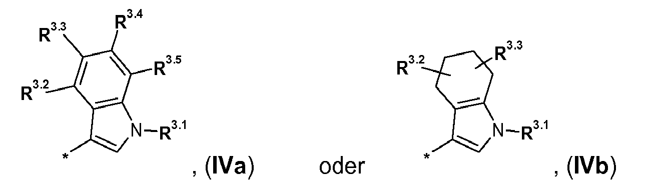
**R**^{3.1} (a) H, (b) -C₁₋₄-Alkylen-**R**^{3.1.1}, -C(O)-C₁₋₃-Alkyl,
**R**^{3.1.1} (a) H, (b) -C(O)-O-**R**^{3.1.1.1}, -O-**R**^{3.1.1.1}, -N(**R**^{3.1.1.1})₂, (c) eine 5- bis 7-gliedrige Heterocyclylgruppe, die an einem Kohlenstoffatom mit einem oder zwei Resten **R**^{3.1.1.2} substituiert sein kann, (d) eine -C(O)-Heterocyclylgruppe, die an einem Kohlenstoffatom mit einem oder zwei Resten **R**^{3.1.1.2} substituiert sein kann,
**R**^{3.1.1.1} unabhängig voneinander H, C₁₋₆-Alkyl und
**R**^{3.1.1.2} unabhängig voneinander H, F, C₁₋₃-Alkyl,
**R**^{3.2} (a) H, (b) Halogen, -CN-, -O-C₁₋₃-Alkyl, C₁₋₃-Alkyl, -C(O)-O-**R**^{3.2.1}, (c) eine C₁₋₃-Alkyl- oder C₁₋₃-Alkyl-O-Gruppe, in der jede Methylengruppe mit bis zu zwei Fluoratomen und jede Methylgruppe mit bis zu drei Fluoratomen substituiert ist,
**R**^{3.2.1} H, C₁₋₆-Alkyl,
**R**^{3.3} (a) H, (b) Halogen, -CN, -O-C₁₋₃-Alkyl, C₁₋₃-Alkyl, -C(O)-O-**R**^{3.3.1}, (c) eine C₁₋₃-Alkyl- oder C₁₋₃-Alkyl-O-Gruppe, in der jede Methylengruppe mit bis zu zwei Fluoratomen und jede Methylgruppe mit bis zu drei Fluoratomen substituiert ist,
**R**^{3.3.1} H, C₁₋₆-Alkyl,
**R**^{3.4} (a) H, (b) Halogen, -CN, -O-C₁₋₃-Alkyl, C₁₋₃-Alkyl, -C(O)-O-**R**^{3.4.1}, (c) eine C₁₋₃-Alkyl- oder C₁₋₃-Alkyl-O-Gruppe, in der jede Methylengruppe mit bis zu zwei Fluoratomen und jede Methylgruppe mit bis zu drei Fluoratomen substituiert ist,
**R**^{3.4.1} H, C₁₋₆-Alkyl,
**R**^{3.5} (a) H, (b) Halogen, -CN, -O-C₁₋₃-Alkyl, C₁₋₃-Alkyl, -C(O)-O-**R**^{3.5.1}, (c) eine C₁₋₃-Alkyl- oder C₁₋₃-Alkyl-O-Gruppe, in der jede Methylengruppe mit bis zu zwei Fluoratomen und jede Methylgruppe mit bis zu drei Fluoratomen substituiert ist,
**R**^{3.5.1} H, C₁₋₆-Alkyl bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

7. Verbindungen der allgemeinen Formel **I** gemäß Anspruch 1, in denen **U, V, X, Y, R**¹ und **R**² wie in Anspruch 1 definiert sind und
**R**³ eine Gruppe ausgewählt aus 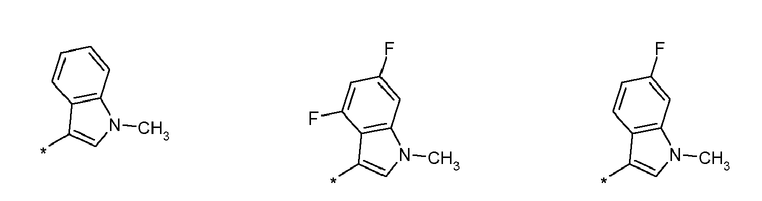 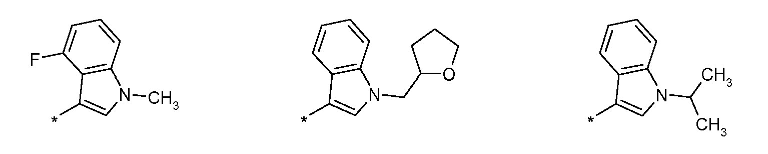 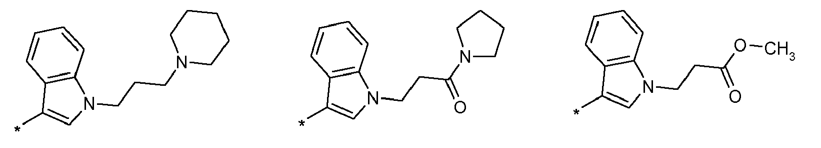 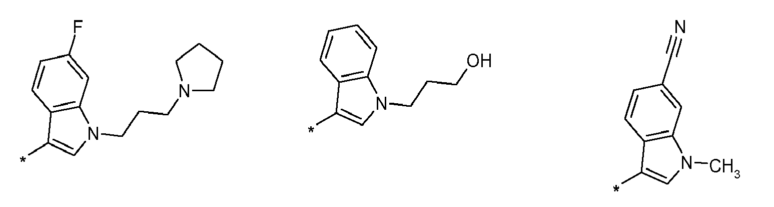 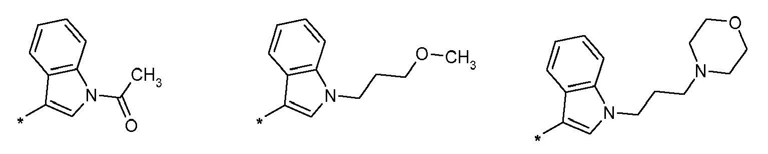 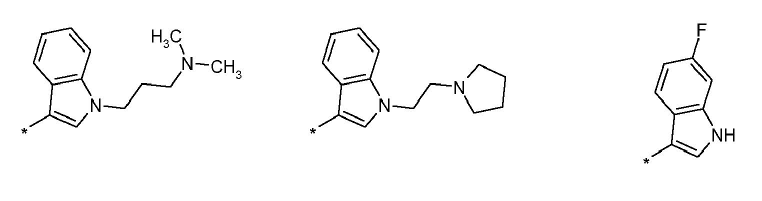 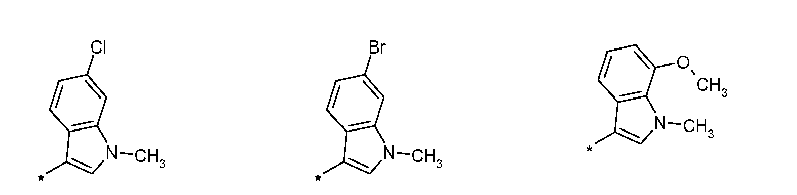 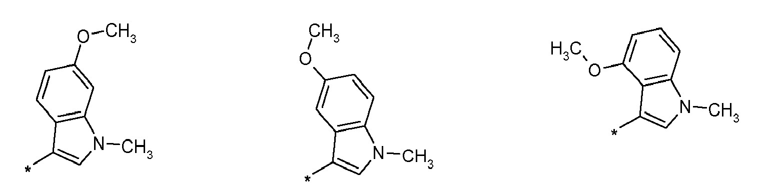 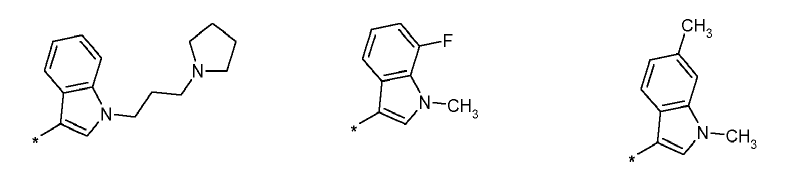 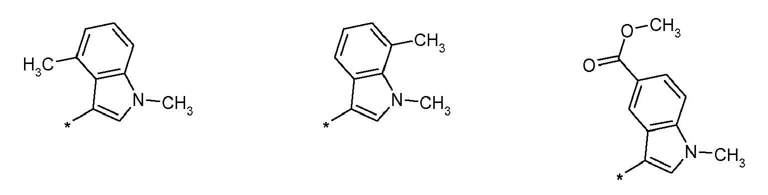 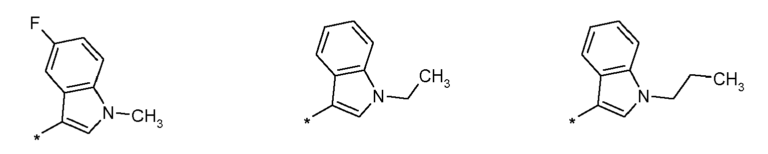 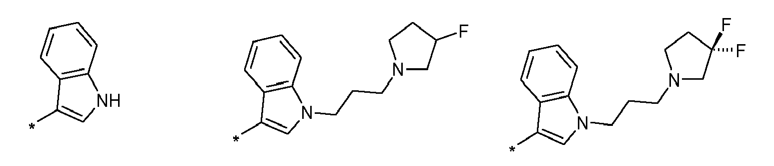 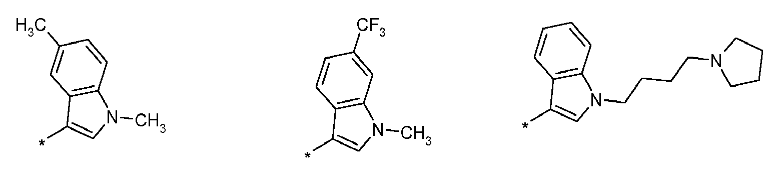 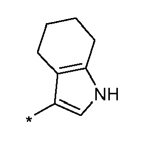 bedeutet, deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

8. Verbindungen der allgemeinen Formel **I** gemäß Anspruch 1, in denen **Y, R¹, R²** und **R**³ wie voranstehend unter der ersten Ausführungsform definiert sind und **U-V-X** eine Gruppe ausgewählt aus -N=N-(CH)=, -N=(C-**R**⁴)-N=, -N=(C-**R**⁴)-(CH)=, -(N-Oxid)=(C-**R**⁴)-(CH)=, -(CH)=N-N=, -(CH)=N-(CH)=, -(CH)=(C-**R**⁴)-N=, -(CH)=(C-**R**⁴)-(N-Oxid)=, -(CH)=(C-**R**⁴)-(CH)= und
**R**⁴ H, -N**R**^{4.1}**R**^{4.2} oder -O-C₁₋₃-Alkyl,
**R**^{4.1} H oder C₁₋₆-Alkyl, **R**^{4.2} H oder -SO₂-C₁₋₃-Alkyl bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

9. Verbindungen der allgemeinen Formel I gemäß Anspruch 1, in der
**R**¹ eine Gruppe ausgewählt aus 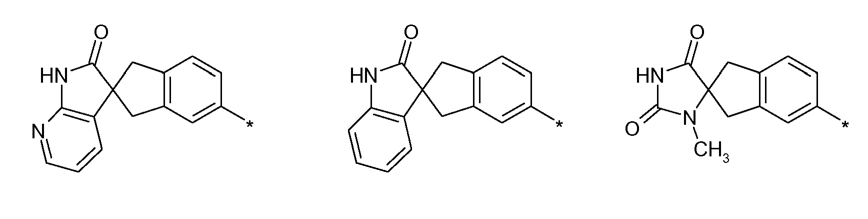 und
**R**² H oder **R**¹ und **R**² zusammen mit dem Stickstoffatom, an das sie gebunden sind, eine Gruppe ausgewählt aus 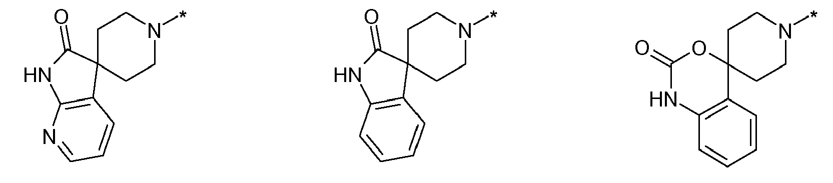 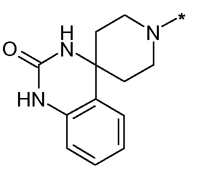
**R**³ eine Gruppe ausgewählt aus 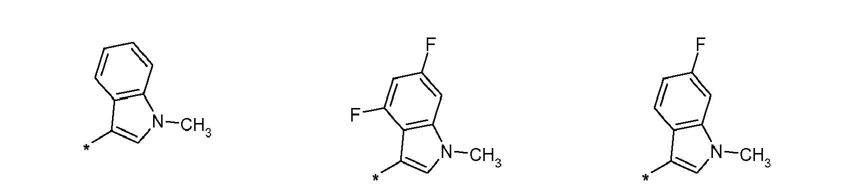 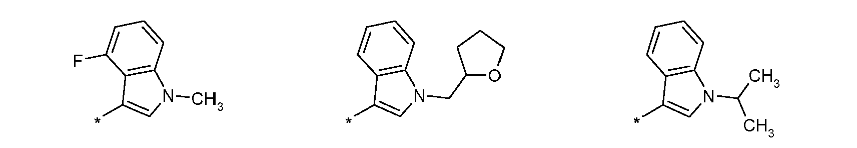 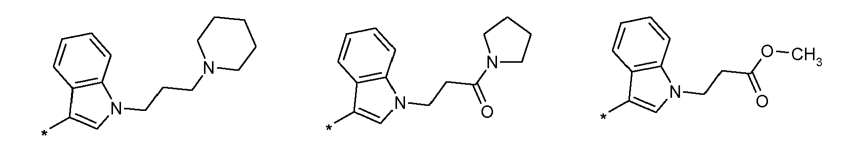 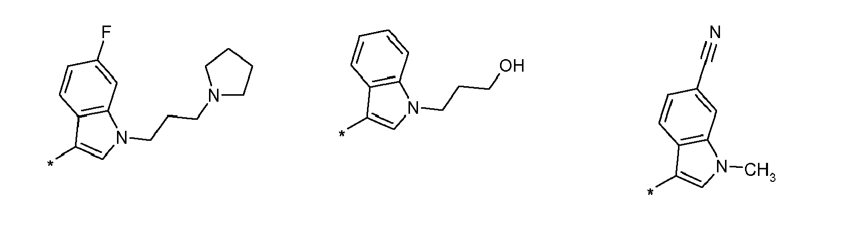 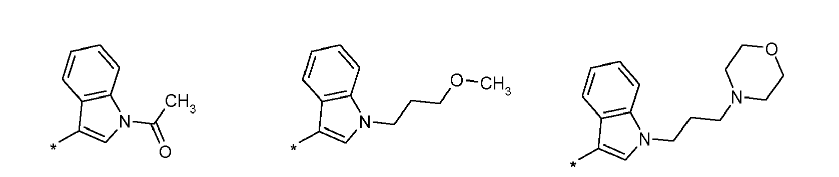 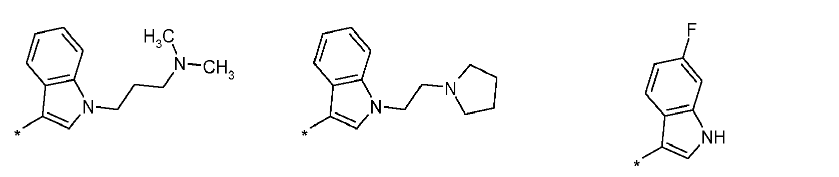 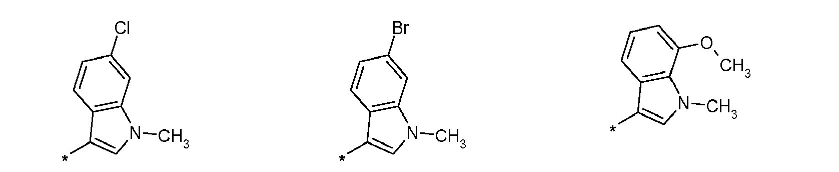 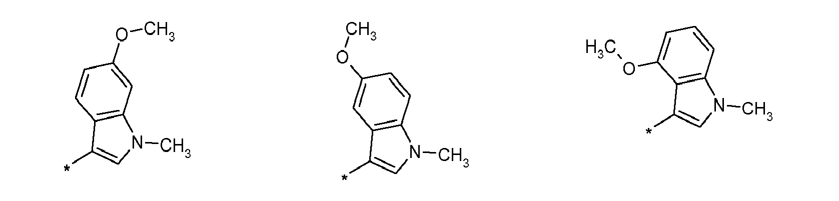 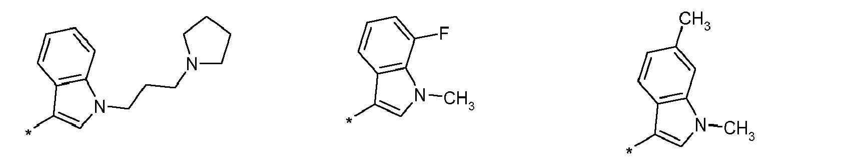 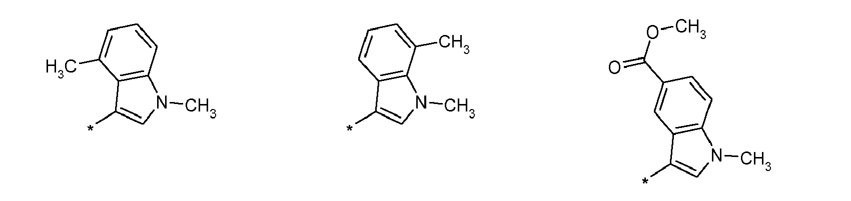 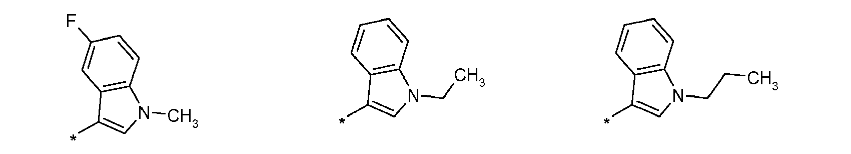 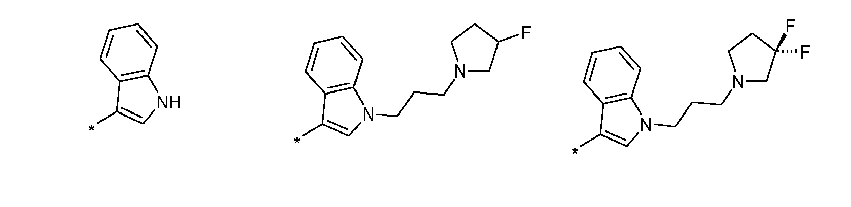 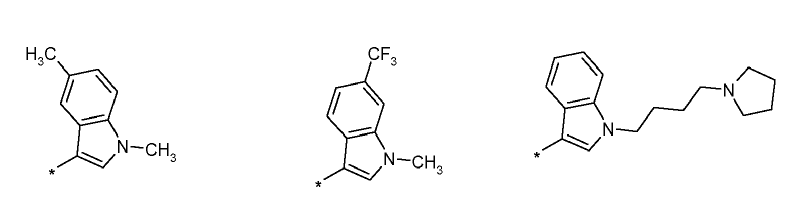 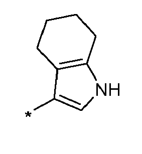 und der Ring 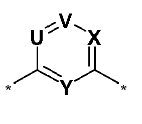 eine Gruppe ausgewählt aus 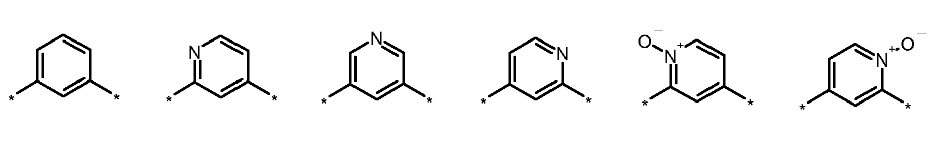 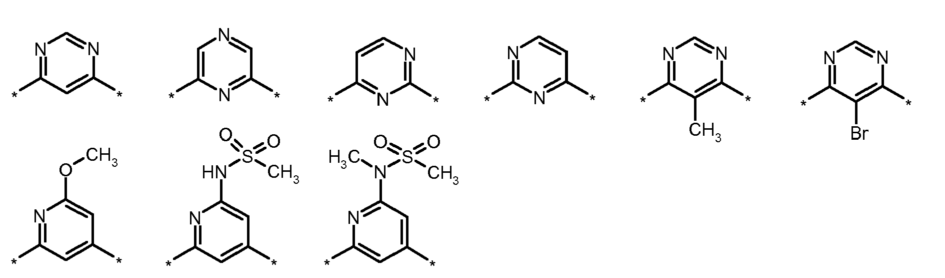 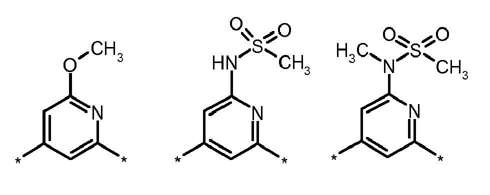 bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

10. Folgende Verbindungen der allgemeinen Formel **I** gemäß Anspruch 1:
| **Nr.** | **Struktur** |
|---|---|
| (1) | 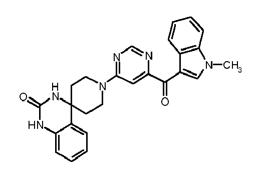 |
| (2) | 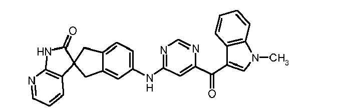 |
| (3) | 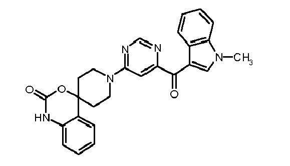 |
deren Enantiomere, deren Diastereomere, deren Hydrate, deren Gemische und deren Salze sowie die Hydrate der Salze, insbesondere deren physiologisch verträgliche Salze mit anorganischen oder organischen Säuren oder Basen.

11. Physiologisch verträgliche Salze der Verbindungen nach einem der Ansprüche 1 bis 10 mit anorganischen oder organischen Säuren oder Basen.

12. Arzneimittel, enthaltend eine Verbindung nach einem der Ansprüche 1 bis 10 oder ein physiologisch verträgliches Salz gemäß Anspruch 11 neben gegebenenfalls einem oder mehreren inerten Trägerstoffen und/oder Verdünnungsmitteln.

13. Verwendung einer Verbindung nach einem der Ansprüche 1 bis 11 zur Herstellung eines Arzneimittels zur akuten und prophylaktischen Behandlung von Kopfschmerzen, insbesondere Migräne- bzw. Cluster-Kopfschmerz.

14. Verwendung einer Verbindung nach einem der Ansprüche 1 bis 11 zur Herstellung eines Arzneimittels zur Behandlung von nicht insulinabhängigem Diabetes mellitus ("NIDDM"), des complex regional pain syndrome (CRPS1), cardiovaskulärer Erkrankungen, Morphintoleranz, Clostritiumtoxin-bedingten Durchfallerkrankungen, Erkrankungen der Haut, insbesondere thermische und strahlenbedingte Hautschäden inklusive Sonnenbrand, entzündlicher Erkrankungen, z.B. entzündlicher Gelenkerkrankungen (Arthritis), neurogenen Entzündungen der oralen Mucosa, entzündlichen Lungenerkrankungen, allergischer Rhinitis, Asthma, Erkrankungen, die mit einer überschießenden Gefäßerweiterung und **dadurch** bedingter verringerter Gewebedurchblutung einhergehen, z.B. Schock und Sepsis, zur Linderung von Schmerzzuständen, oder zur präventiven oder akut-therapeutisch Behandlung der Symptomatik menopausaler, durch Gefäßerweiterung und erhöhten Blutfluss verursachter Hitzewallungen östrogendefizienter Frauen sowie hormonbehandelter Prostatakarzinompatienten.

15. Verfahren zur Herstellung eines Arzneimittels gemäß Anspruch 12, **dadurch gekennzeichnet, dass** auf nichtchemischem Weg eine Verbindung nach einem der Ansprüche 1 bis 11 in einen oder mehrere inerte Trägerstoffe und/oder Verdünnungsmittel eingearbeitet wird.
